(19) Europäisches Patentamt / European Patent Office / Office européen des brevets

(11) EP 2 365 007 B1

(12) **EUROPEAN PATENT SPECIFICATION**

(45) Date of publication and mention of the grant of the patent:
**02.09.2015 Bulletin 2015/36**

(21) Application number: **11003742.1**

(22) Date of filing: **16.04.2004**

(51) Int Cl.:
*C08F 8/00* (2006.01) *C08F 8/34* (2006.01)
*A61L 31/16* (2006.01) *A61L 31/18* (2006.01)
*A61K 9/00* (2006.01) *A61F 2/82* (2013.01)
*A61K 9/51* (2006.01)

(54) **Magnetically controllable drug and gene delivery stents**

Stents mit magnetisch steuerbarer Arzneimittel- und Genabgabe

Endoprothèse pour administration de gènes et de médicaments contrôlable magnétiquement

(84) Designated Contracting States:
**AT BE BG CH CY CZ DE DK EE ES FI FR GB GR HU IE IT LI LU MC NL PL PT RO SE SI SK TR**

(30) Priority: **16.04.2003 US 463505 P**
**17.02.2004 US 545127 P**
**20.02.2004 US 546233 P**

(43) Date of publication of application:
**14.09.2011 Bulletin 2011/37**

(62) Document number(s) of the earlier application(s) in accordance with Art. 76 EPC:
**04750256.2 / 1 620 039**

(73) Proprietors:
- **The Children's Hospital of Philadelphia Philadelphia, PA 19104 (US)**
- **Drexel University Philadelphia, PA 19104 (US)**

(72) Inventors:
- **Levy, Robert J. Merion Station, PA 19066 (US)**
- **Chorny, Michael Philadelphia, PA 19116 (US)**
- **Fischbein, Ilia Philadelphia, PA 19116 (US)**
- **Alferiev, Ivan Clementon, NJ 08021 (US)**
- **Friedman, Gennady Richboro, PA 18954 (US)**
- **Barbee, Kenneth A. Philadelphia, PA 19119 (US)**
- **Yellen, Benjamin B. Northbrook, IL 60062 (US)**
- **Forbes, Zachary G. Philadelphia, PA 19106 (US)**

(74) Representative: **Katzameyer, Michael et al**
**v. Bezold & Partner**
**Patentanwälte - PartG mbB**
**Akademiestraße 7**
**80799 München (DE)**

(56) References cited:
**EP-A2- 0 207 495** **US-A- 5 916 539**
**US-A1- 2001 014 448**

- **CHEN G ET AL: "Gradient micropattern immobilization of EGF to investigate the effect of artificial juxtacrine stimulation", BIOMATERIALS, ELSEVIER SCIENCE PUBLISHERS BV., BARKING, GB, vol. 22, no. 18, 1 September 2001 (2001-09-01), pages 2453-2457, XP004273570, ISSN: 0142-9612, DOI: 10.1016/S0142-9612(00)00432-4**
- **Jill K. Veilleux, Lise W. Duran: "Covalent immobilization of bio-molecules to preactivated surfaces", , 1 March 1996 (1996-03-01), XP002674441, Retrieved from the Internet: URL: http://www.ivdtechnology.com/article/c ovalent-immobilization-bio-molecules-preac tivated-surfaces [retrieved on 2012-04-23]**
- **AMOS RA ET AL DONALD L WISE: "Biomaterial Surface Modification Using Photochemical Coupling Technology", 1 January 1995 (1995-01-01), ENCYCLOPEDIC HANDBOOK OF BIOMATERIALS AND BIOENGINEERING, NEW YORK [U.A.] : DEKKER, US, PAGE(S) 895 - 926, XP008151086, ISBN: 0-8247-9593-8 * the whole document ***

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 2 365 007 B1

## Description

BACKGROUND OF THE INVENTION

1. FIELD OF INVENTION

The present invention provides water-soluble photo-activatable polymers and implantable devices comprising the same. This invention also relates to surface modifications and more particularly to immobilization of molecules to surfaces by photochemical coupling. Further, this invention also relates to a magnetizable implantable devices utilizing magnetizable or magnetic particles acting as carriers of biomaterial, biomolecules and cells.

2. DESCRIPTION OF RELATED ART

Stents are commonly used in a variety of biomedical applications. For example, stents are routinely implanted in patients to keep blood vessels open in the coronary arteries, to keep the esophagus from closing due to strictures of cancer, to keep the ureters open for maintenance of kidney drainage, and to keep the bile duct open in patients with pancreatic cancer. Such stents are usually inserted percutaneously under radiological guidance.

Stents comprise a tube made of metal or polymer, in a wide range of physiologically appropriate diameters and lengths, which are inserted into a vessel or passage to keep the lumen open and prevent closure due to a stricture or external compression. General stent design varies in the number of intersections and interstrut area, the in-strut configuration, and the metal-to-artery ratio. The two different expansion principles for stents are balloon-expansion and self-expansion, and the design types can be categorized into five types: ring, tubular, multi-design, coil, and mesh (Regar et al. Br. Med. Bull. 2001 59:227-48; Hehrlein et al. Basic Res. Cardiol. 2002 97:417-23; Gershlick et al. Atherosclerosis 2002 160:259-71; Garas et al. Pharmacology and Therapeutics 2001 92:165-78).

Stents have been routinely used over the last ten years in percutaneous transluminal coronary angioplasty (PTCA), a procedure for the treatment of severe, symptomatic coronary stenosis (Garas, S.M. et al. Pharmacology and Therapeutics 2001 92:165-178). The PTCA procedure was first introduced in the 1970s as an alternative to coronary-artery bypass surgery for the clearing of coronary vessels blocked by plaque. PTCA has proven to be a much less invasive procedure, with patients able to return to work the week following the procedure, as opposed to the lengthy hospital stay required with bypass surgery (Fricker, J. Drug Discovery Today 2001 6:1135-7). Stents are used extensively in PTCA procedures due to their unique ability to master a major complication of balloon angioplasty ((sub) acute vessel closure), and a superior long-term outcome in comparison to balloon angioplasty (Regar et al. Br. Med. Bull. 2001 59:227-48).

However, in-stent restenosis (the re-closing of the vessel) remains a major limitation, particularly in coronary stenting. Restenosis is generally considered a local vascular manifestation of the biological response to injury. The injury as a result of catheter insertion consists of denudation of the intima (endothelium) and stretching of the media (smooth muscle). The wound-healing reaction consists of an inflammatory phase, a granulation phase, and a remodeling phase. The inflammation is characterized by growth factor and platelet activation, the granulation by smooth muscle cell and fibroblast migration and proliferation into the injured area, and the remodeling phase by proteoglycan and collagen synthesis, replacing early fibronectin as the major component of extracellular matrix. Coronary stents comprise mechanical scaffolding that almost completely eliminates recoil and remodeling. However, neo-intimal growth or proliferation is still a problem. Neo-intimal proliferation occurs principally at the site of the primary lesion within the first 6 months after implantation, a major checkpoint for patient health post-surgery (Regar et al. Br. Med. Bull. 2001 59:227-48). Neo-intima forms during the first week after PTCA and the progress is well under way after 4 weeks, with continued progression over the following months (Hehrlein et al. Basic Re. Cardiol. 2002 97:417-23). This neo-intima is an accumulation of smooth muscle cells within a proteoglycan matrix that narrows the previously enlarged lumen. Its formation is triggered by a series of molecular events including leukocyte infiltration, platelet activation, smooth muscle cell expansion, extracellular matrix elaboration, and re-endothelialization (Regar et al. Br. Med. Bull. 2001 59:227-48).

Three major drug techniques under consideration for the prevention of restenosis are (i) prevention of thrombus formation; (ii) prevention of vascular recoil and remodeling; and (iii) prevention of inflammation and cell proliferation (Garas et al. Pharmacology and Therapeutics 2001 92:165-78). *In vitro* and *in vivo* animal model experimentation has shown promise in all three categories, mainly in antiproliferation treatments. However, clinical success has been limited (Garas et al. Pharmacology and Therapeutics 200192:165-78), primarily due to systemic toxicity.

Local drug delivery provides limited systemic release, thereby reducing the risk of systemic toxicity. Techniques for local drug delivery that have been described include, but are not limited to, direct coating of the stent with drug, coating of the stent with a drug-containing biodegradable polymer, and hydrogel/drug coating. Biodegradable stents have also been described (Regar et al. Br. Med. Bull. 2001 59:227-48; Hehrlein et al. Basic Res. Cardiol. 2002 97:417-23; Gershlick et al. Atherosclerosis 2002 160:259-71; Garas et al. Pharmacology and Therapeutics 2001 92:165-78; Schwartz

et al. Circulation 2002 106:1867-73; Fricker, J. Drug Discovery Today 2001 6:1135-7). Problems with these technologies, however, include the inflammatory response generated due to large polymer concentrations, the inability to deliver effective concentrations, one-time dosage limitations, and, in the case of the biodegradable stent, mechanical compromise. An additional concern with the polymer-coated drug-eluting stents is limitation of the growth of the cell layer necessary to cover the stent and prevent the bare metal from coming in long contact with the blood, thereby leading to clot formation (Schwartz et al. Circulation 2002 106:1867-73; Fricker, J. Drug Discovery Today 2001 6:1135-7).

The ability to apply forces on magnetic particles with external magnetic fields has been harnessed in various biomedical applications including prosthetics (Herr, H. J. of Rehab. Res. and Devel. 2002 39(3):11-12), targeted drug delivery (Goodwin, S. J. of Magnetism and Magnetic Materials 1999 194:209-217) and antiangiogenesis strategies (Liu et al. J. of Magnetism and Magnetic Materials 2001 225:209-217; Sheng et al. J. of Magnetism and Magnetic Materials 1999 194:167-175). U.S. Patent 4,247,406 describes an intravascularly-administrable, magnetically-localizable biodegradable carrier comprising microspheres formed from an amino acid polymer matrix containing magnetic particles embedded within the matrix for targeted delivery of chemotherapeutic agents to cancer patients. Microspheres with magnetic particles, which are suggested to enhance binding of a carrier to the receptors of capillary endothelial cells when under the influence of a suitable magnetic field, are also described in U.S. Patent 5,129,877.

U.S. Patents 6,375,606; 6,315,709; 6,296,604; and 6,364,823 describe methods and compositions for treating vascular defects, and in particular aneurysms with a mixture of biocompatible polymer material, a biocompatible solvent, an adhesive and preferably magnetic particles to control delivery of the mixture. In these methods, a magnetic coil or ferrofluid is delivered via catheter into the aneurysm. This magnetic device is shaped, delivered, steered and held in place using external magnetic fields and/or gradients. This magnetic device attracts the mixture to the vascular defect wherein it forms an embolus in the defect thereby occluding the defect.

A model for inducing highly localized phase transformations at defined locations in the vascular system by applying 1) external uniform magnetic fields to an injected superparamagnetic colloidal fluid for the purpose of magnetization and 2) using implants with patterned magnetic material to create high magnetic field gradients was described (Forbes et al. Abstract and Poster Presentation at the 6th Annual New Jersey Symposium on Biomaterials, October 17-18, 2002, Somerset, NJ). The use of these magnetizable implants in drug delivery was also described previously by authors Z. Forbes, B.B. Yellen, G. Friedman, and K. Barbee (IEEE Trans. Magn. 39(5): 3372-3377 (2003)).

In general, methods and devices which have been proposed for delivery of magnetizable drug or agent-containing magnetic carrier to specific locations in the body rely upon a single source of magnetic field to both magnetize the carriers and to pull them by magnetic force to the specific location. These single magnetic fields for attracting magnetically susceptible therapeutic agents can be applied by sources external to the body, or by an internal implant as described by Chen in U.S. Patent 5,921,244.

Single source capture methods, however, are at odds with the underlying physics of magnetic particle capture, which depends on the simultaneous imposition of very strong far-reaching magnetic fields and strong spatial magnetic field gradients. The purpose of the far-reaching field is to increase the magnetic moment of individual drug-containing particles in the vicinity of the field to the point of magnetic saturation. Far-penetrating fields are most typically generated with large magnetic sources. However, the force on a magnetized particle also depends on production of strong magnetic field gradients, which are most easily generated with very small magnetic sources. Thus, the ability to simultaneously produce far-penetrating magnetic fields that have strong magnetic field gradients is very difficult to accomplish with a single source. For this reason, Chen teaches use of relatively large electromagnets implanted in tissue beds to attract magnetic fluid circulating within the blood vessels that are relatively far away, which is a less effective method for capturing magnetic carriers. The present invention further differs from previous techniques in that the goal is to deliver therapeutic agents to a desired tissue site without obstructing flow through the blood vessel.

In general, nanoparticles have been very ineffective vehicles for gene delivery, with expression levels below those seen with naked DNA. Thus, there has been relatively little progress with DNA incorporation into biodegradable sustained release particles. Also, problems encountered in gene therapy include slow accumulation and low concentration of gene vector in target tissues.

Nanoparticles formed from biodegradable polymers have been used to carry active molecules to sites in the body where the therapeutic effect is required (see Quintanar-Guerrero et al., Preparation techniques and mechanisms of formation of biodegradable nanoparticles from preformed polymers. Drug Dev Ind Pharm 1998; 24:1113-28; and Kumar MNR. Nano and microparticles as controlled drug delivery devices. J Pharm Pharmaceut Sci 2000; 3:234-58). Quintanar-Guerrero et al. describe various techniques available to prepare biodegradable nanoparticles from polymers such as for example, emulsification-solvent evaporation, solvent displacement, salting-out, and emulsification diffusion. In general, such nanoparticles have limited loading capacity for most hydrophilic drugs and also are not efficient in cases where rapid accumulation of active molecules is required at their target sites.

Various studies were conducted to improve delivery of a biomaterial such as viruses (e.g., adenovirus) and plasmid DNA by physical means such as an application of a magnetic field to a vector including magnetically responsive solid phases, which are micro-to nanometer sized particles or aggregates thereof (see Plank et al., Enhancing and

targeting nucleic acid delivery by magnetic force. Expert Opin Biol Ther. 2003; 3:745-58 (Plank I thereafter); Plank et al., The magnetofection method: using magnetic force to enhance gene delivery. Biol Chem. 2003; 384:737-47 (Plank II thereafter); Scherer et al. Magnetofection: enhancing and targeting gene delivery by magnetic force in vitro and in vivo. Gene Ther. 2002; 9:102-9).

Ito et al. describe application of magnetic granules (0.1-0.5 microns) as carriers for anti-cancer drugs administered orally in local targeting chemotherapy of esophageal cancer ( see Magnetic Granules: A Novel System for Specific Drug Delivery to Esophageal Mucosa in Oral Administration. Int'l. J. of Pharmaceutics, 61 (1990), pp. 109-117). Compositions described by Ito et al. were not made as colloidal particles, and magnetic granules used therein were not stabilized.

U.S. Patent No. 5,916,539 to Pilgrimm describes superparamagnetic particles useful in medicine for destroying tumors, increasing immunity and diagnosing conditions. The patent describes aggregates of superparamagnetic single-domain particles bearing on its surface chemically bound organic substances for further binding of active substances such as antigens, antibodies, haptens, protein A, protein G, endotoxin-binding proteis, lectins, and selectins.

Arias et al. describes an anionic polymerization procedure for preparing colloidal nanoparticles consisting of a magnetic core and a biodegradable polymeric shell wherein the polymerization medium was magnetite suspension in HCl solution (see Synthesis and Characterization of Poly(ethyl-2-cyanoacrylate) Nanoparticles with a Magnetic Core. J of Controlled Release 77 (2001), pp. 309-321).

Gómez-Lopera et al. describes preparation of colloidal particles formed by a magnetite nucleus and a biodegradable poly(DL-lactide) polymer coating by a double emulsion method, wherein aqueous suspension of magnetite particles was used to prepare an emulsion with the polymer (see Synthesis and Characterization of Spherical Magnetite/Biodegradable Polymer Composite Particles. J. of Colloid and Interface Science 240, 40-47 (2001)). It is significant that the magnetite in the cited studies was not incorporated as an organic suspension, resulting in its poor incorporation in the particle.

Plank et al. describe superparamagnetic iron oxide nanoparticles manufactured with polyelectrolyte surface coatings such as poly(ehylenimine) (PEI) and polylysine further associated with gene vectors by salt induced colloid aggregation (Magnetofection: enhancing and targeting gene delivery with superparamagnetic nanoparticles and magnetic fields. J Liposome Res. 2003; 13:29-32 (Plank III thereafter)). (See also Plank II, Scherer et al., Magnetofection: enhancing and targeting gene delivery by magnetic force in vitro and in vivo. Gene Ther. 2002; 9:102-9).

The same research group further used magnetic beads in combination with PEI and pDNA as a model of a non-viral vector mediated gene expression system for transfection of cells (see Krotz et al., Magnetofection potentiates gene delivery to cultured endothelial cells. J Vasc Res. 2003; 40(5):425-434) and delivery of antisense oligonucleotides in a catheter-based coronary angioplastic therapy for occlusive cardiovascular disease (see Krotz et al., Magnetofection-A highly efficient tool for antisense oligonucleotide delivery in vitro and in vivo. Mol Ther. 2003; 7:700-10). The magnetic beads used by this research group lack the concept of sustained release and increased colloidal stability achievable with biodegradable polymer-based particles. Moreover, the reported results show a considerable extent of cell toxicity caused by nanoparticulate formulations (see Plank II, supra). The PEI coating stability and a possible aggregation in biological fluids have not been examined, but might potentially be a concern in these formulations.

Müller et al. studied cytotoxicity of poly(lactide), poly(lactide-co-glucolide), poly(styrene) and solid lipid particles loaded with magnetite. No attempts to incorporate magnetite as a stable organic dispersion were described (see Cytotoxicity of Magnetite-Loaded Polylactide, Polylactide/Glycolide Particles and Solid Lipid Nanoparticles. Int'l. J. of Pharmaceutics 138 (1996) 85-94). Further, the possibility of loading the particles with a drug has not been examined.

Igartua et al. describes encapsulation of magnetite particles stabilized by oleic acid in solid lipid nanoparticles (see Development and Characterization of Solid Lipid Nanoparticles Loaded with Magnetite. Int'l. J. of Pharmaceutics 233 (2002) 149-157). The authors did not address using polymer as a matrix and presented no results on drug loading in such particles.

De Cuyper et al. describes magnetoliposomes which are phospholipid bilayer coated magnetite particles prepared by adsorption of sonicated phospholipids onto magnetite stabilized by lauric acid in an aqueous solution (see Magnetoliposomes. Formation and structural characterization. Eur Biophys J 1988; 15:311-319). Such liposomes are too small to be effectively manipulated by magnetic field. Although, ability to bind drug have not been studied, the liposomes prepared by this method have limited capacity for drug substances since they can be loaded only by surface adsorption.

Messai et al. describe poly (lactic acid)-based particles ((PLA nanoparticles) surface modified by electrostatic adsorption of PEI, wherein PEI is associated with DNA (see Elaboration of Poly(ethyleneimine) Coated Poly(D, L-lactic acid) Particles. Effect of Ionic Strength on the Surface Properties and DNA Binding Capabilities. Colloids and Surfaces B: Biointerfaces 32 (2003), pp. 293-305). PEI adsorbed onto PLA nanoparticles does not provide a stable coating and readily dissociates into the external medium upon dilution.

Sullivan et al. describe gene delivery scaffolds based on DNA plasmid condensation with colloidal gold/PEI conjugates (see Development of a Novel Gene Delivery Scaffold Utilizing Colloidal Gold-Polyethylenimine Conjugates

for DNA Condensation. Gene Therapy (2003) 10, 1882-1890). Although, such conjugates when used as a vehicle for gene delivery exhibit improved size stability when compared to PEI alone, they cannot be targeted by magnetic field and lack sustained release properties.

Further, a common shortcoming of implantable medical devices or surfaces is recognition of these devices by an organism as foreign objects followed by inflammation or even a rejection of such devices. Surface modification science concentrates on finding a better interface between a living tissue and a solid matrix. It is known to use various coatings to impart desirable properties to implantable surfaces. Such coatings are based on polymers and may include biologically active materials. Challenges in preparing such coatings include attaching biologically active materials to inert surfaces.

One of the techniques for derivatizing inert surfaces is photochemical coupling (see Amos et al., Biomaterial surface modification using photochemical coupling technology. In: Wise D L, Trantolo D J, Altobelli D E, Yaszemski M J, Gresser J D, Schwartz E R editors. Encyclopedic handbook of biomaterials and bioengineering, Part A: materials. New York: Marcel Dekker Inc., 1995, p. 895-926). Photo-cross-linking chemistry based on organic solvents is well established (see Wetzels et al., Photoimmobilization of poly(N-vinylpyrrolidone) as a means to improve haemocompatibility of polyurethane biomaterials, Biomaterials 1999, 20, 1879-87; McClung et al., Lysine-derivatized polyurethane as a clot lysing surface: conversion of absorbed plasminogen to plasmin and clot lysis in vitro, Biomaterials 2001, 22, 1919-24; Aldenhoff et al., Photo-immobilization of dipyridamole (PERSANTIN®) at the surface of polyurethane biomaterials: reduction of in vitro trombogenicity, Biomaterials 1997,18, 167-72; Kuijpens et al., Immobilization of theophylline on medical-grade polyurethane inhibits surface-induced activation of blood platelets, J. Am. Chem. Soc. 1995, 117, 8691-8697). It is not known to use adsorption from aqueous solutions for application of photo-cross-linkers onto a polymer surface.

Aryl ketones (e.g., benzophenone or acetophenone derivatives) and aryl azides are known as photo-activatable cross-linkers suitable for covalent binding to virtually any type of polymer surface as described by Amos et al.) Upon irradiation with long-wave UV (at about 350 nm), benzophenone residues form energy-rich excited triplet species, which then insert into carbon-hydrogen bonds (C-H bonds) of a polymeric surface by abstraction of hydrogen atoms from C-H bonds and form new carbon-carbon bonds (C-C bonds), resulting in covalent binding of benzophenone residues onto the polymeric surface (see FIG. 1).

U.S. Patent No. 5,071,909 to Pappin et al. discloses a method for immobilizing proteins or peptides onto a membrane by formation of a polymeric network, which entraps the protein or peptide.

U.S. Patent Nos. 3,959,078, 5,512,329 and 5,741,551 to Guire et al. disclose covalent bonding of polymeric molecules to surfaces through external activation. This approach is used to bind fibronectin peptide to a polystyrene surface by a photo-reaction between the peptide and a surface having a photo-activatable group.

U. S. Patent No. 5,637,460 to Swan et al. discloses attaching a target molecule (synthetic polymers, carbohydrates, proteins, lipids, nucleic acids, etc.) to a surface by using photo-activatable groups.

Chen et al. (in Biomaterials, Elsevier Science Publishers BV., Barking GB, vol. 22, no. 18, pages 2453-2457 (2001)) disclose an azidophenyl-derivatized polyallylamine for the immobilization of a biomolecule to a polystyrene plate.

Despite the foregoing developments, there is a need in the art for alternative means of delivery of biomaterial.

## BRIEF SUMMARY OF THE INVENTION

The invention provides a water-soluble photo-activatable polymer comprising a polymer precursor which is poly(allylamine) and the following groups covalently attached to the poly(allylamine):

(a) a photo-activatable group that upon irradiation is capable of forming a covalent bond between the water-soluble photo-activatable polymer and a matrix having at least one carbon;
(b) a thiol-reactive group;
(c) a hydrophilic group, wherein the hydrophilic group is present in an amount sufficient to make the water-soluble photo-activatable polymer soluble in water;

wherein the thiol-reactive group is a 2-pyridyldithio group, and the hydrophilic group is a carboxy group.

In certain embodiments, the photo-activatable group is a member selected from the group consisting of an aryl ketone and an aryl azide. In certain embodiments, the aryl ketone is a member selected from the group consisting of benzophenone and acetophenone.

In certain embodiments, the water-soluble polymer is represented by a formula:

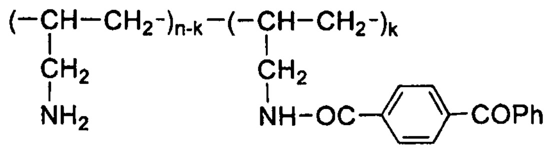

wherein n is 50 to 2000 and k is 10 to 1000.

In certain embodiments, the water-soluble polymer is represented by a formula:

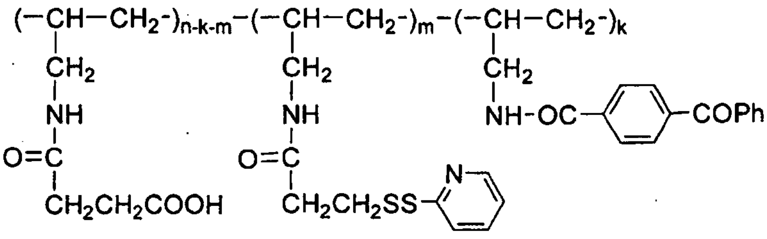

wherein n is 50 to 2000, k is 10 to 1000, and m is 10 to 1000.

Further provided is a process for producing the water-soluble photo-activatable polymer of the invention, the process comprising:

providing a polymer precursor comprising a plurality of reactive groups and a plurality of hydrophilic groups;
providing a photo-activatable reagent; and
reacting a first portion of the reactive groups and/or hydrophilic groups with the photo-activatable reagent.

In certain embodiments of the process, the first portion is from about 1% to about 50% of the reactive groups and/or hydrophilic groups.

In certain embodiments, the first portion is about 20% of the reactive groups and/or hydrophilic groups.

In certain embodiments, the process for producing the water-soluble photo-activatable polymer of the invention further comprises:

providing a reagent comprising a 2-pyridyldithio group, and
reacting a second portion of the reactive groups and/or hydrophilic groups with the reagent to obtain the water-soluble polymer.

In certain embodiments, a sum of the first portion and the second portion is from about 60% to about 80%.

Further provided is a composition comprising a monomolecular layer of the water-soluble photo-activatable polymer of the invention and a matrix having at least one carbon, wherein the monomolecular layer is covalently attached to the matrix by a covalent bond between the photo-activatable group and the at least one carbon.

In certain embodiments, the composition further comprises a biomaterial having a plurality of active groups, wherein the biomaterial is covalently attached to the monomolecular layer by covalent bonding between the active groups and reactive groups.

In certain embodiments of the composition, at least one of the active groups is a member selected from the group consisting of amine, carboxyl, hydroxyl, thiol, phenol, imidazole, and indole. Preferably, the at least one of the active groups comprise thiol.

In certain embodiments of the composition, the biomaterial is a member selected from the group consisting of an antibody, a viral vector, a growth factor, a bioactive polypeptide, a polynucleotide coding for the bioactive polypeptide, a cell regulatory small molecule, a peptide, a protein, an oligonucleotide, a gene therapy agent, a gene transfection vector, a receptor, a cell, a drug, a drug delivering agent, nitric oxide, an antimicrobial agent, an antibiotic, an antimitotic, dimethyl sulfoxide, an antisecretory agent, an anti-cancer chemotherapeutic agent, steroidal and non-steroidal anti-inflammatories, hormones, an extracellular matrix, a free radical scavenger, an iron chelator, an antioxidant, an imaging agent, and a radiotherapeutic agent. Preferably, the biomaterial is an anti-knob antibody, an adenovirus, a D1 domain of the Coxsackie-adenovirus receptor, insulin, an angiogenic peptide, an antiangiogenic peptide, avidin, biotin, IgG, protein A, transferrin, and a receptor for transferrin.

In certain embodiments of the composition, the matrix is a member selected from a group consisting of polyurethane, polyester, polylactic acid, polyglycolic acid, poly(lactide-co-glycolide), poly(ε-caprolactone), polyethyleneimine, polystyrene, polyamide, rubber, silicone rubber, polyacrylonitrile, polyacrylate, and polymetacrylate, poly(alpha-hydroxy

acid), poly(dioxanone), poly(orthoester), poly(ether-ester), poly(lactone), polytetrafluoroethylene, organosilane, mixtures thereof and copolymers thereof.

In certain embodiments of the composition, the matrix further comprises a superparamagnetic agent. Preferably, the superparamagnetic agent is a member selected from the group consisting of magnetite and maghemite nanocrystals as such, as aggregates or as dispersion in polymer from the list above.

In certain embodiments of the composition, the matrix is an implantable device. Preferably, the implantable device comprises at least one member selected from the group consisting of polyurethane, polyester, polylactic acid, poly(lactide-co-glycolide), poly(ε-caprolactone), polyethyleneimine, polystyrene, polyamide, rubber, silicone rubber, polyacrylonitrile, polyacrylate, polymetacrylate, polytetrafluoroethylene, organosilane, mixtures thereof and copolymers thereof.

In certain embodiments of the composition, the matrix is a particle having a diameter of about 5 nm to about 10 microns. Preferably, the particle comprises at least one member selected from the group consisting of polylactic acid, poly(lactide-co-glycolide), poly(ε-caprolactone), polyethyleneimine, mixtures thereof and copolymers thereof.

Further provided is a method of making the composition of the invention, the method comprising:

providing the matrix having at least one carbon;
providing an aqueous solution of the water-soluble photo-activatable polymer having the photo-activatable group and the 2-pyridyldithio group;
contacting the matrix with the aqueous solution; and
photo-activating the photo-activatable group by irradiation to covalently attach the water-soluble polymer via the photo-activatable group to the matrix and thereby forming the monomolecular layer of the composition.

In certain embodiments of the method of making the composition of the invention, the irradiation is performed at a wavelength from about 190 to about 900 nm. Preferably, the irradiation is performed at a wavelength of 280 to 360 nm.

Additionally, certain embodiments of the method further comprise providing a biomaterial having a plurality of active groups and reacting the plurality of active groups with the water-soluble photo-activatable polymer to covalently attach the biomaterial to the matrix. Further, the invention provides an implantable device comprising:

a surface having a magnetizable compound and
a magnetic carrier comprising at least one of a superparamagnetic agent, magnetic-field responsive agent, and a coated magnetic-field responsive agent, wherein the magnetic carrier is attracted to the magnetizable compound by a magnetic force, provided that the magnetic carrier is a surface modified particle comprising the water-soluble photo-activated polymer of the invention.

In certain embodiments, the magnetizable compound comprises at least one of cobalt, iron, nickel, oxides and alloys thereof. In certain embodiments, the magnetizable compound comprises magnetic particles such as for example, ferromagnetic particle provided that they are biodegradable and biocompatible.

In certain embodiments, the surface modified particle comprises a water-soluble photo-activated polymer and a biomolecule covalently attached to the water-soluble photo-activated polymer.

BRIEF DESCRIPTION OF SEVERAL VIEWS OF THE DRAWINGS

The invention will be described in conjunction with the following drawings in which like reference numerals designate like elements and wherein:

Figs. 1A and 1B are diagrams of an exemplary embodiment of a device of the present invention implanted in an artery. In this embodiment, the device comprises a magnetizable stent wherein segments of magnetizable compound have been electro-deposited as a mesh-like structure on some of the struts of the stent. Darkened bars depict the segments of the magnetizable compound which has been deposited on the stent. As shown in Fig.1A, blood flow through the artery brings a therapeutic agent in a magnetic carrier in proximity with the implanted stent. As shown in Fig.1B, as blood flows through the stent, the therapeutic agent in the magnetic carrier is attracted to the segments of the stent with the magnetizable compound.
Figs. 2A and 2B are scanning electron microscopy images of bead capture obtained using a wire mesh electroplated with the magnetizable compound Cobalt Nickel alloy. Fig. 2A shows the mesh before exposure to the magnetic beads while Fig. 2B shows the mesh after exposure to 2.8-micron magnetic beads.
Fig. 3 is a schematic illustration of the magnetically targeted delivery.
Fig. 4A is a schematic illustration of a top view of micro-channels having walls seeded with magnetic particles.
Fig. 4B is a chart depicting a flow of micro-particle solutions through micro-channels shown in Fig. 4A.

Fig. 5A is a bar graph showing transfection efficacy of magnetic nanoparticles examined as a function of magnetic field exposure in the absence of magnetic field exposure.

Fig. 5B is a bar graph showing transfection efficacy of magnetic nanoparticles examined as a function of magnetic field exposure in the presence of magnetic field exposure.

Fig. 6 is a graph showing cell toxicity of magnetic NP determined in BAEC cells by WST-1 assay following 4 hour incubation at 37° C as a function of the concentration of particle-associated PEI or free PEI used as a control.

Fig. 7 is a graph showing the effect of the organic phase composition on the size of freshly prepared particles and size stability observed 7 days after particles' preparation.

Fig 8 is a graph showing the extent and stability of PEI association with magnetic NP as a function of the organic phase composition.

Fig. 9 is a scheme showing a preferred method of making a particle comprising the matrix-forming agent, polyelectrolyte-amphiphilic agent adduct, the coated magnetic field-responsive agent and the biomaterial.

Fig. 10 is a reaction scheme (Scheme 1) illustrating binding of benzophenone cross-linkers to polymers having C-H bonds.

Fig. 11 is a reaction scheme (Scheme 2) demonstrating synthesis of polymeric multipoint benzophenone modifiers.

Fig.12 is a reaction scheme (Scheme 3) depicting immobilization of thiol-containing proteins on a polymeric surface.

## DETAILED DESCRIPTION OF THE INVENTION

The invention was i.a. driven by the desire to develop a composition and a method for surface modification of inert surfaces useful for implantation, which would permit attachment of molecular therapeutics such as proteins, genes, vectors, or cells and avoid using organic solvents that can potentially damage both the surface and molecular therapeutics. Moreover, utilizing therapeutic potential of site-specific therapy (SST) with custom synthesized stent surfaces and heart valve leaflets, the present photochemical approach will permit surface activation of a broad range of existing medical device configurations.

The inventors have discovered that biomaterial can be covalently attached to surfaces having at least one carbon by utilizing a water-soluble photo-activatable polymer of the invention which functions as a multipoint polymeric cross-linker, wherein one function of the cross-linker is to photoimmobilize the water-soluble polymer to a desired surface and another function is to attach the desired biomaterial.

The water-soluble photo-activatable polymer of the invention comprises:

a polymer precursor which is poly(allylamine) and the following groups covalently attached to the poly(allylamine):

(a) a photo-activatable group that upon irradiation is capable of forming a covalent bond between the water-soluble photo-activatable polymer and a matrix having at least one carbon;

(b) a thiol-reactive group;

(c) a hydrophilic group, wherein the hydrophilic group is present in an amount sufficient to make the water-soluble photo-activatable polymer soluble in water;

wherein the thiol-reactive group is a 2-pyridyldithio group, and the hydrophilic group is a carboxy group.

The specific chemistry used herein has distinct advantages since it involves: 1) aqueous based exposures, thus removing any risk of damaging surfaces that could be susceptible to organic solvent damage, and 2) addition of reactive groups, e.g., PDT groups, thus enabling sulfhydryl chemistry approaches for attaching linking proteins and peptides, such as antibodies or receptor fragments.

A reaction between a thiol-reactive group (2-pyridyldithio, maleimide, etc.) attached to one protein molecule with a thiol group of another protein molecule (or other biomolecule) is a widely used for preparation of protein conjugates (See Greg T. Hermanson, Bioconjugate Techniques, Academic Press, San Diego 1996). Reaction of a thiol group with most of thiol-reactive groups (particularly 2-pyridyldithio group) is very selective and fast in aqueous media at mild conditions. Proteins can be thiolated using a partial reduction of disulfide bridges or via thiolation of lysine residues with a variety of reagents (see Hermanson, pp. 57-70). The reaction of thiolated proteins with polymeric surfaces containing thiol-reactive groups would be ideal for the immobilization of proteins on polymeric supports. At the same time, there is no method for providing polymeric surfaces with such thiol-reactive groups. The present invention can efficiently solve this problem.

One of the reasons the surface-attachment of 2-pyridyldithio-groups (PDT-groups) to polymers via benzophenone photo-cross-linkers is not obvious is that PDT-groups are rather unstable and may not survive conditions of photo-immobilization wherein active, energy-rich species appear.

The term "photo-activatable group" used herein denotes chemical groups capable of generating active species

such as free radicals, nitrenes, carbenes and excited states of ketons upon absorption of external electromagnetic or kinetic (thermal) energy. These groups may be chosen to be responsive to various portions of the electromagnetic spectrum, i.e., the groups responsive to ultraviolet, visible and infrared portions of the spectrum. The preferred photo-activatable groups of the invention are benzophenones, acetophenones and aryl azides. Upon excitation, photo-activatable groups are capable of covalent attachment to surfaces comprising at least one carbon such as polymers.

The water-soluble photo-activatable polymer of the invention may have one or more photo-activatable groups. In certain embodiments, the water-soluble photo-activatable polymers have at least one photo-activatable group per molecule. Preferably, the water-soluble photo-activatable polymers have a plurality of photo-activatable groups per molecule. More preferably, photo-activatable groups modify at least 0.1% of monomeric units of a polymer precursor, even more preferably at least 1%, and most preferably from about 20 to about 50 %.

The irradiation source can be any source known in the art capable of emitting the light having a wavelength absorbable by the photo-activatable group of the invention. A UV-lamp is preferred when the benzophenone is used as the photo-activatable group.

The term "water-soluble polymer" as used in this disclosure means that the water-soluble photo-activatable polymer of the invention can be diluted with water to at least 1 wt% and preferably to at least 0.1 wt% to form a single phase at a temperature of 20°C, provided that water is essentially free of an organic co-solvent.

The terms "surface" or "matrix" as used interchangeably herein mean a surface having at least one carbon. In a preferred embodiment of the invention, the surface is a polymeric matrix. Other surfaces such as organosylated materials (i.e., organosylated metals) can also be used in the present invention.

The surface contemplated by the present invention can have any shape or form suitable for variety of purposes such as for example a delivery of a biomaterial to an organism. In that, the surface can be an existing medical implant such as a stent or a cardiovascular valve, which can be covered with the composition of the invention. Also, the surface can be first modified with either the water-soluble polymer of the invention or the composition of the invention and then molded into the desired shape. Moreover, the surface can be in a form of polymeric particles.

Medical devices appropriate for the gene delivery system in the present invention include, but are not limited to, heart valves, wire sutures, temporary joint replacements and urinary dilators. Other suitable medical devices for this invention include orthopedic implants such as joint prostheses, screws, nails, nuts, bolts, plates, rods, pins, wires, inserters, osteoports, halo systems and other orthopedic devices used for stabilization or fixation of spinal and long bone fractures or disarticulations. Other devices may include non-orthopedic devices, temporary placements and permanent implants, such as tracheostomy devices, jejunostomy and gastrostomy tubes, intraurethral and other genitourinary implants, stylets, dilators, stents, vascular clips and filters, pacemakers, wire guides and access ports of subcutaneously implanted vascular catheters.

The polymeric matrix of the invention can be biodegradable and non-biodegradable. Non-limiting examples of the polymeric matrix used in the invention are poly(urethane), poly(ester), poly(lactic acid), poly(glycolic acid), poly(lactide-co-glycolide), poly($\varepsilon$-caprolactone), poly(ethyleneimine), poly(styrene), poly(amide), rubber, silicone rubber, poly(acrylonitrile), poly(acrylate), poly(metacrylate), poly(alpha-hydroxy acid), poly(dioxanone), poly(orthoester), poly(ether-ester), poly(lactone), mixtures thereof and copolymers thereof.

In certain embodiments of the composition, the matrix further comprises a superparamagnetic agent. Preferably, the superparamagnetic agent is a member selected from the group consisting of magnetite and maghemite nanocrystals.

The water-soluble photo-activatable polymer of the invention comprises a polymer precursor and the following groups covalently attached to the polymeric precursor: the photo-activatable group as described above, a reactive group, and a hydrophilic group.

The polymeric precursor of the water-soluble photo-activatable polymer of the invention can be prepared by using methods known in the art from a polymer (biodegradable or a non-biodegradable) comprising reactive groups, and hydrophilic groups, which is then modified to contain photo-activatable groups. Also, it can be prepared by polymerization of monomeric blocks containing the above groups. In the invention, the polymeric precursor is polyallylamine. In certain embodiments of the invention, the polyallylamine has a molecular weight of about 200 KDa to about 5 KDa. In the preferred embodiment, the molecular weight is from 70 KDa to 15 KDa.

The reactive group of the water-soluble photo-activatable polymer of the invention is a chemical group adapted to covalently react with a biomaterial. According to the invention, the reactive group is a thiol-reactive group, namely a 2-pyridyldithio group.

The hydrophilic group of the water-soluble photo-activatable polymer of the invention is present in an amount sufficient to make the water-soluble photo-activatable polymer soluble in water. In the invention, the hydrophilic group is a carboxy group.

In certain embodiments of the invention, the photo-activatable group is a member selected from the group consisting of an aryl ketone and an aryl azide. Preferably, the aryl ketone is a member selected from the group consisting of benzophenone and acetophenone.

In one embodiment of the invention, the water-soluble polymer is polyallylamine based benzophenone (PAA-BzPh) and is represented by a formula:

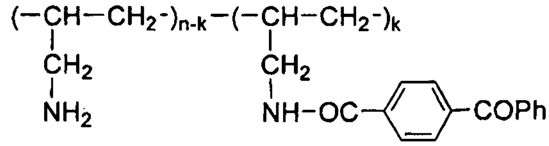

wherein n is 50 to 2000 and k is 10 to 1000.

In another embodiment of the invention, the water-soluble polymer is polyallylamine based benzophenone further modified to contain 2-pyridyldithio groups (PDT-BzPh) and is represented by a formula:

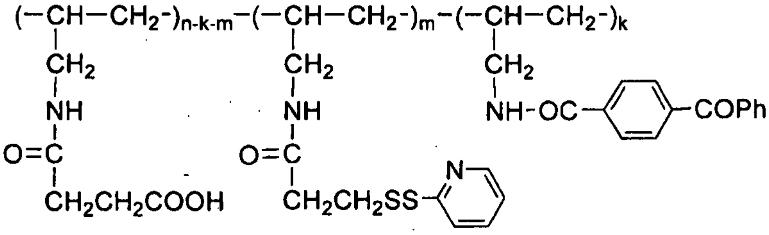

wherein n is 50 to 2000, k is 10 to 1000, and m is 10 to 1000.

The water-soluble polymer the invention can be prepared by using radical polymerization of a mixture of three types of monomers (e.g., acrylamide-based), each containing only one of the groups, as shown below:

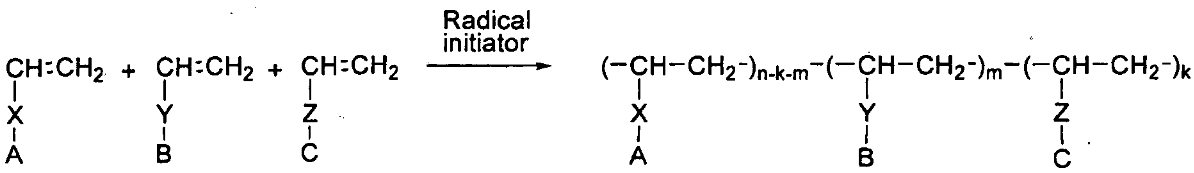


X,Y and Z are suitable spacers
A = photo-activatable group, B = hydrophilic group, C = reactive group

The ratio between these groups in the final product can be controlled by changing the ratio of monomers. Conditions for polymerization are known to persons skilled in the art. Additionally, each monomer can bear two, or all the three types of the groups. Other types of polymerization or polycondensation known in the art can also be used.

Upon excitation of photo-activatable groups, the water-soluble polymer of the invention is covalently bonded to the surface by forming a monomolecular layer on the surface.

The term "layer" used herein means a contiguous and non-contiguous deposit formed by a covalent bonding of polymers of the invention to the surface. Preferably, the layer is highly homogeneous and high purity in that it consists essentially of the water-soluble polymer of the invention.

Further provided is a composition of matter comprising a monomolecular layer of the water-soluble photo-activatable polymer of the invention and a matrix having at least one carbon, wherein the monomolecular layer is covalently attached to the matrix by a covalent bond between the photo-activatable group and the at least one carbon.

In certain embodiments, the composition further comprises a biomaterial having a plurality of active groups, wherein the biomaterial is covalently attached to the monomolecular layer by covalent bonding between the active groups and reactive groups.

In certain embodiments of the composition, at least one of the active groups is a member selected from the group consisting of amine, carboxyl, hydroxyl, thiol, phenol, imidazole, and indole. Preferably, the at least one of the active groups comprise thiol.

Also disclosed herein is a method for delivery of a biomaterial to a cell, the process comprising: providing the composition of the invention comprising the monomolecular layer of the water-soluble photo-activatable polymer covalently attached to the matrix; providing a biomaterial having a plurality of active groups, wherein the biomaterial is covalently attached to the monomolecular layer by covalent bonding between the active groups and the reactive groups; and administering the matrix to the cell and thereby delivering the biomaterial. Preferably, the biomaterial is a protein, a D1 domain of the Coxsackie-adenovirus receptor or an antibody specifically bound to a nucleic acid, wherein said

antibody is bound to the matrix. Still more preferred, the protein, the D1 domain of the Coxsackie-adenovirus receptor, and the antibody are a thiol modified. The composition can then be used to deliver the biomaterial to the interior of a cell in need of, for example, gene therapy.

Antibodies specific for non-viral vectors or nucleic acid may require use of a transfection agent to enhance administration of nucleic acid. The transfection agent is a cationic macromolecule that is positively charged, comprises two or more art-recognized modular units (e.g., amino acid residues, fatty acid moieties, or polymer repeating units), and is preferably capable of forming supermolecular structures (*e.g.*, aggregates, liposomes or micelles) at high concentration in aqueous solution or suspension. Among the types of cationic macromolecules that can be used are cationic lipid and polycationic polypeptides.

The amount of the transfection agent to be used when transfecting cells can be calculated based on nucleic acid content of the composition. The capacity of the medium comprising or containing the transfection agent can also affect the amount of transfection agent to be used. When the antibody of the transfection agent is immobilized on a matrix, the amount of cationic macromolecule and DNA that can be complexed with the antibody can be limited by the physical requirements of the metal support. For example, rigidity, flexibility and chemical reactivity may influence the amount of transfection agent used. Such vectors have included retroviral, adenovirus, adeno-associated viral vectors and herpes viral vectors. Cells can be infected with viral vectors by known methods.

Further provided is a method of making the composition of the invention, the method comprising: providing the matrix having at least one carbon; providing an aqueous solution of the water-soluble photo-activatable polymer having the photo-activatable group and the reactive group; and photo-activating the photo-activatable group by irradiation to covalently attach the water-soluble polymer via the photo-activatable group to the matrix and thereby forming the monomolecular layer of the composition.

In certain embodiments of the method of making the composition of the invention, the irradiation is performed at a wavelength from about 190 to about 900 nm. Preferably, the irradiation is performed at a wavelength of 280 to 360 nm.

Additionally, certain embodiments of the method further comprise providing a biomaterial having a plurality of active groups and reacting the plurality of active groups with the water-soluble photo-activatable polymer to covalently attach the biomaterial to the matrix.

PHOTOCHEMICAL MODIFICATION OF MICRO-AND NANOPARTICLES

The water-soluble photo-activatable polymers of the invention obtained as described above can be bound to the surface of pre-formed micro- or nanoparticles (MP and NP, respectively) or during the particle formation process to form modified particles capable of reacting with the biomaterial.

Advantageously, in the preparation of biodegradable and non-biodegradable polymeric micro- and nanoparticles, the water-soluble photo-activatable polymer of the invention (e.g., BzPh-PDT) not only makes the surface reactive towards the thiol-containing biomolecules, but also prevents the aggregation of the particles and thus stabilizes the particle suspension in the suspending medium.

Non-limiting examples of uses of particles modified with PDT-BzPh are various applications for delivery of therapeuticals to the body. For example, injectable nanoparticles can be used to either provide an intravenously applied sustained delivery system for proteins and peptides potentially targeted to a site of interest, or if injected into a specific location such as a tumor, or the myocardium to provide, for example, sustained local presence of therapeutic peptides and proteins. This approach would be desirable in a broad range of applications covering virtually every conceivable disease process.

Depending on the specific application of the particulate formulation stating demands on its properties such as particle size and composition, the particles can be prepared by one of the existing methods (See Couvreur P et al., Nanoparticles: preparation and characterization. In: Benita S, Editor, Microencapsulation. Methods and industrial applications. vol. 73. New York: Marcel Dekker, 1996. pp. 183-211; Kumar MNR. Nano- and microparticles as controlled drug delivery devices. J Pharm Pharmaceut Sci 2000; 3:234-58).

Generally, particle formation may be accomplished by a number of methods of which the most widely used are emulsification-polymerization and polymer precipitation techniques. The former may be accomplished by *in situ* polymerization of monomers either in aqueous solution or emulsified in aqueous phase (namely, emulsification-polymerization). Alternatively, methods exploiting pre-formed biocompatible polymers, usually of poly(ester) and poly(anhydrid) families, can be used to form particles by polymer precipitation methods. The most popular methods are emulsification-solvent evaporation, emulsification-diffusion and nanoprecipitation methods (See Quintanar-Guerrero et al., Preparation techniques and mechanisms of formation of biodegradable nanoparticles from preformed polymers. Drug Dev Ind Pharm 1998; 24:1113-28). The latter methods are based on emulsifying an organic solution of a polymer with or without drug in an aqueous phase in presence of a stabilizer substance (e.g. Poloxamer 188, polyvinyl alcohol, etc.) achieved either by external energy input or through spontaneous diffusion of water-miscible solvents with subsequent solvent elimination to form solid particle dispersion.

While both emulsification-polymerization and polymer precipitation are applicable for preparing matrix-type particles (spheres), some of these methods with appropriate modifications can be used for producing core-shell type vesicles (capsules). The drug substance can either be encapsulated (dissolved or dispersed in the polymeric matrix of a sphere or dissolved in the liquid core of a capsule) or adsorbed/chemically bound to the particle surface. The latter approach where a substance is attached to the surface of a preformed particle has the advantage of avoiding harsh conditions (extreme pH, exposure to organic solvents or elevated temperatures) employed for the particle formulation. Covalent association of the drug with the particle surface employing biodegradable chemical bonds (e.g. by disulfide linking) provides an alternative that achieves both controlled and site-specific release of the drug.

An additional degree of site-specificity may be achieved by rendering the particles magnetic by inclusion of magnetite/maghemite nanocrystals in the polymeric matrix (e.g. Ito R, Machida Y, Sannan T, Nagai TU-hwscsaBTW-B-Gddaaca. Magnetic granules: a novel system for specific drug delivery to esophageal mucosa in oral administration. Int J Pharm 1990; 61:109-117). This modification allows for concentrating the particles at their target tissue using magnetic field, thereby increasing their therapeutic efficacy and minimizing the potential formulation toxicity.

Also disclosed is a process for delivery of a biomaterial to a cell or an organism, the process comprising (1) providing the composition of the invention as a monomolecular layer and a matrix having at least one carbon, wherein the monomolecular layer is covalently attached to the matrix by a covalent bond between the photo-activatable group and the at least one carbon, (2) providing a biomaterial having a plurality of active groups, wherein the biomaterial is covalently attached to the monomolecular layer by covalent bonding between the active groups and the reactive groups; and (3) administering the matrix to the cell or an organism.

Amounts of the biomaterial may vary depending on the purpose of delivery, e.g., prophylactic, diagnostic, therapeutic, etc. and on the nature of the biomaterial involved.

In certain embodiments, the biomaterial delivered by this method is at least one of a low molecular weight therapeutical, a protein, a nucleic acid or a therapeutic virus, of which the latter three can be bound either directly, or through an affinity ligand, such as specific antibody or D1 domain of the Coxsackie-adenovirus receptor in the case of the adenovirus.

In the following, the present application describes specific implantable devices as well as methods for using these devices in the targeted delivery of therapeutic agents in a body or a subject. As far as these devices comprise the characterizing features of claim 28, including the presence of a water-soluble photo-activatable polymer as defined in claim 1 and above, they are embodiments of the present invention.

By the term "therapeutic agent" as used herein is meant to include, but is not limited to, drugs, imaging agents such as radionuclides, cells, gene therapy agents such as vectors and viruses, and proteins and peptides. The therapeutical agent can be a biomolecule as defined below.

In one embodiment, devices of the present invention comprise a structural supporting implant of biocompatible metal or polymer. Implants of the present invention further comprise a magnetizable compound. In one embodiment, the magnetizable compound is segmented on the structural supporting implant to provide regions of high field gradients distributed throughout the implant. In another embodiment, the magnetizable compound is uniformly coated on the structural supporting implant, and the magnetized state of the coating is either locally recorded to provide regions of high field gradients distributed throughout the implant or uniformly magnetized. In yet another embodiment, the implant itself comprises the magnetizable compound..

By magnetizable compound, as used herein, it is meant a material that conducts magnetic flux strongly. Examples of magnetizable compounds useful in the implants of the present invention include, but are not limited to, cobalt, iron, iron oxides, nickel, and rare earth magnetic materials and various alloys. In one embodiment, the magnetizable compound is magnetized only in the presence of externally applied fields. Examples of these types of magnetizable compound include, but are not limited to, superparamagnets and soft ferromagnets. In other embodiment, magnetizable compounds known as ferromagnets, which can be permanently magnetized, are used.

In one embodiment, the structurally supporting implant itself is made entirely from alloys of magnetizable compounds.

In another embodiment, a layer of magnetizable compound is placed on the structural supporting implant as a coating with a thickness ranging from about 2 nanometers to about 200 microns or in segments. When placed on as segments of magnetizable compound, it is preferred that these segments extend through and around the structural supporting implant from one end of the implant to the other. Similar arrangements include, but are not limited to, multiple rings extending over the implant and a mesh-like structure surrounding the implant such as depicted in Figures 1 and 2.

The magnetizable compound can be applied to the structural supporting implant by various methods including, but not limited to, electro-deposition, evaporation and sputtering, and by chemical reactions.

Magnetic carriers may not always be uniformly attracted to implants that are simply coated with magnetic material. This is due to the fact that magnetic domains in such coatings are hard to control. Thus, several means of patterning the magnetic coating to control domain patterns in the devices of the present invention can be used.

One such means involves laser-assisted electrodeposition of alloys of magnetic metals such as Co, Ni and Fe.

In this method, the implant, preferably a stent is used as a cathode during the deposition and a voltage slightly below electroplating threshold is applied. Magnetic material can then be electroplated only in those spots that are exposed to a focused laser beam.

Another method for patterning of the implant with the magnetizable compound involves the use of magnetic nanoparticles and nanorods separately prepared. These may either be purchased or made by electroplating into nanotemplates. Magnetic nanoparticles are then deposited onto the implant through a process called dielectrophoresis. In this process the implant is placed into an aqueous solution containing magnetic nanoparticles in between two insulated electrodes. Application of a relatively high frequency (100 KHz-1MHz) electric field creates strong, high frequency electric field gradients on the implant that attracts the nanoparticles.

Another method for patterning the implant involves recording of magnetic domain pattern on the implant using methods closely related to those that are employed in magnetic information storage devices. One such approach involves laser assisted thermomagnetic recording. In this method, the implant is first uniformly magnetized by a strong external field. Subsequently selected spots are heated by a laser in the presence of a reversed magnetic field. The strength of the reversed field is sufficient to reverse magnetization of heated spots, but insufficient to reverse magnetization of unheated spots. Spots that have been heated by a laser are then magnetized in opposition to the rest of the implant magnetization.

In one embodiment of the device of the present invention, the implant is a stent comprising a metallic tube such as, but not limited to, a corrugated stainless steel tube, coated with a magnetizable compound such as cobalt, iron, iron oxides, nickel, or other rare earth magnetic materials or alloys, followed by a passivating layer of a biocompatible material. In this embodiment, it is preferred that the stent maintain its capability for balloon expansion and complete mechanical integrity so that it is useful not only in selective targeting of therapeutic agents, but also in keeping the lumen into which it is inserted open. Accordingly, this stent-based delivery system, when used in procedures such as PTCA, preserves the beneficial properties of stents (preventing vascular recoil and remodeling) while delivering therapeutic agents, preferably drugs or radionuclides, that inhibit or measure intimal thickening, respectively.

The segments of magnetizable compound coated on the stent provide the stent with the capability of attracting arterially injected magnetic carriers including, but not limited to magnetic particles, magnetic liposomes and ferrofluids encapsulating or attached to the therapeutic agent, over the entire stent, thus distributing the therapeutic agent over the entire stent so that possible clogging of the artery and/or stent by deposited therapeutic agent is decreased.

In an alternative embodiment of the device of the present invention, the implant comprises a plurality of biocompatible metal or polymer beads, spikes or pellets wherein at least one segment of each bead, spike or pellet comprises the magnetizable compound. Various means for implanting such a device into the selected site are known and can be selected by one of skill in the art based upon the site of implantation. For example, for implantation into a blood vessel wall, the device may be delivered via a catheter-based system. In this embodiment, it is preferred that the catheter be equipped with a balloon coated with the plurality of implants so that upon expansion of the balloon the implants are lodged into the blood vessel. Alternatively, the balloon can be coated with a plurality of implants which have been modified to further comprise a specific receptor for the endothelial lining which bind to the endothelial lining upon contact. In another embodiment, the device can be implanted by injection of the plurality of magnetizable beads administered to the site of interest and lodged into the tissue by application of external magnetic field gradients. Alternatively, a device of the present invention comprising a plurality of implants can be injected directly into the site of interest by a means similar to a biopsy needle so as to provide a region of high internal magnetic field that attracts magnetic therapeutic agent-containing particles. In these embodiments, it is preferred that the plurality of implants be scattered over the site of treatment so that the therapeutic agent is dispersed over the treatment site and possible clogging of the artery by deposited agent is decreased.

Also described herein is the use of these devices in the targeted delivery of a therapeutic agent to a selected site in a subject. In this use, a device of the present invention is first implanted into a subject at a selected site. The site of implantation in the subject is selected based upon where targeted treatment is desired and the mode of administration for the therapeutic agent.

For example, for PTCA procedures, the treatment site is a coronary artery at a region of stenosis. The therapeutic agent is preferably a drug that is administered intravascularly to prevent restenosis at this site. The implant, preferably a stent, is thus also implanted in the coronary artery at the site of stenosis. In a preferred embodiment, the device of the present invention is implanted by catheterization in accordance with well-known procedures.

While a primary application of these devices is for treatment of cardiovascular disease and/or restenosis, alternative functions for implants of the present invention are envisioned. These include, but are not limited to, treatment of tumors (benign and malignant), bacterial or viral infections, cysts, internal wounds, and anti-rejection treatments for transplant patients. These treatments can be performed by implantation of the device at the mouth of the site of blood supply, or placement of a device with a plurality of implants within or on the site itself, followed by systemic administration of therapeutic agent attached to or encapsulated in a magnetic carrier such as magnetic particles, magnetic liposomes or a ferrofluid.

There are numerous other potential sites of implantation envisioned for a magnetizable/magnetic device of the present invention. It is important to note that in all of these cases, a magnetic carrier such as magnetic particles, magnetic liposomes or a ferrofluid is carrying or bound with the therapeutic agent. These include, but are in no way limited to, the lymphatic system for treatment of swollen or infected glands, and damaged or occluded vessels; in the bile ducts for pancreatic cancer patients; in the ureter or urethra for kidney drainage or kidney/bladder infections; in or on the surface of the larynx, trachea, or lung surface for treatment of respiratory disorders or cancers with the use of an inhalable solution of magnetic particles bound with drug. Further, a device of the present invention placed within the esophagus could be used to capture magnetic particles contained within a viscous creeping solution for treatment of cancers or infection.

In addition, devices of the present invention surgically placed within the brain for local delivery of a therapeutic agent could be used to capture magnetic particles, preferably nanoparticles, with lipid-soluble or other permeability-enhancing coatings to allow targeting of intra-arterial chemotherapeutics. While intra-arterial chemotherapeutics are already practiced in various manners to treat brain tumors, magnetic targeting of such treatments may limit healthy neural tissue exposure to chemotherapy while maximizing dosage levels.

The therapeutic agent to be delivered is encapsulated in, attached to, or dispersed in a magnetic carrier. For example, the therapeutic agent may be encapsulated in magnetic particles including, but not limited to, microspheres and nanospheres or magnetic liposomes. Alternatively, the therapeutic agent may be dispersed in a ferrofluid. In embodiments wherein the magnetic carrier involves magnetic particles and/or liposomes, it is preferred that the particles and/or liposomes be less than 10 micrometers in size to prevent clogging of any small arterioles.

It is also preferred that magnetic carriers such as magnetic particles or magnetic liposomes comprise magnetite. Magnetite is a member of the spinel group with the standard formula $Fe_2O_3$ or $Fe_3O_4$. Magnetite particles to be incorporated in the magnetic particles or magnetic liposomes encapsulating the therapeutic agent are preferably around 10 nm in diameter and are dispersed within the magnetic particle or magnetic liposome to account for 10-50% of sphere volume.

In cases where use of a magnetic carrier comprising microspheres or nanospheres for encapsulation of the therapeutic agent is required, the microspheres or nanospheres preferably comprise a biodegradable polymer, such as poly(lactic acid) PLA and/or poly(lactic-co-glycolic acid) PLGA, which cause minimal inflammatory response upon degradation. As will be understood by those of skill in the art upon reading this disclosure, numerous other biodegradable polymers are known, such as polyhydroxybutyrate and elastomeric poly(ester-amide), which may also be used in these microspheres or nanospheres. Ultimate selection of the biodegradable polymer for encapsulation of the drug is based upon desired degradation times, side effects, and drug conjugation.

Selection of a therapeutic agent to be encapsulated within the magnetic carrier such as magnetic particles or magnetic liposomes or dispersed in a magnetic carrier such as ferrofluid and used with the devices of the present invention is dependent upon the use of the device and/or the condition being treated and the site of implantation of the magnetizable device. For example, multiple therapeutic agents have been experimented with and tested for prevention of restenosis following PTCA and any of these can be used with the stents of the present invention. Some examples of such therapeutic agents include, but are not limited to, antiplatelet agents such as aspirin, glycoprotein receptor antagonists, and cilostazol for prevention of thrombus formation by interfering with platelet aggregation; anticoagulants such as heparin, hirudin, and coumadin for prevention of thrombus formation by blocking the coagulation pathway; calcium channel antagonists for reducing vascular recoil and remodeling; growth factor inhibitors, such as trapidil, an inhibitor of PDGF; immunosuppressants such as Rapamycin (Sirolimus; Rapamune®); anti-inflammatory agents; and anti-proliferation agents such as Actinomycin D (Cosmegen®), Estrogen (Estrodiol®), and Paclitaxel (Taxol®). Thus, for PTCA procedures and tumor treatments using a magnetizable stent of the present invention, a preferred therapeutic agent for encapsulation may be Actinomycin D, Rapamycin or Paclitaxel. Other therapeutic agents that can be administered, include, but are not limited to radioactive materials, gene vectors, genetically modified viruses such as retroviruses, and living cells, such as endothelial cells, that are attached to magnetic particles. The ability to attract endothelial cells to the implant will greatly decrease the time it takes to form an endothelial layer on the stent, and may inhibit the growth and migration of smooth muscle cells that are largely responsible for the neo-intimal growth.

Magnetically targeted therapeutic agent delivery achieved through use of a polymer, composition or device of the present invention allows for reduced initial inflammation response often experienced in clinical tests of polymer/drug-coated stents, as the amount of polymer necessary for targeted delivery can be reduced. In addition, since the magnetic vehicles are deliverable via arterial injection, minimally invasive means for delivery of the therapeutic agents at selected times can be used. Thus, in a procedure such as PTCA, a therapeutic agent may not need to be delivered until sufficient time has elapsed for endothelium growth over the stent. Further, multiple doses of the therapeutic agent or combinations of therapeutic agents can be administered.

In some embodiments, the device of the present invention and/or magnetic carrier of the therapeutic agent may require magnetization just prior to, during or following administration of the therapeutic agent. In these embodiments, the device and/or magnetic carrier of the therapeutic agent is magnetized by a magnetic field applied externally to the

subject.

The utility of the devices of the present invention in targeting a therapeutic agent to the site of their implantation is based upon obtaining an attractive magnetic force upon the injected magnetic carrier of the therapeutic agent that overcomes the drag resistance in moving towards the wall. The magnetic force can be optimized by using a device that contains a plurality of magnetic features, producing strong magnetic field gradients that pull the therapeutic agent encapsulated or attached to the magnetic carrier to desired locations on the surface of the implant of the device. The implant of the device is designed to be in direct or proximal contact with the transporting medium for the therapeutic agent so that the therapeutic agent-containing magnetic carrier will be attracted most strongly. For example, in one embodiment as depicted in Figure 1A and 1B, the transporting medium is blood of a blood vessel that has been injected with a colloidal solution of therapeutic agent-containing magnetic particles. In another embodiment, the transporting medium is that of an aerial passageway that has been exposed to a gaseous solution of therapeutic agent-containing magnetic particles in the form of a nasal spray or inhalation. In yet another embodiment, the transporting medium comprises lymphatic or cerebrospinal fluid that has been injected with therapeutic agent-containing magnetic particles that are attracted to a device of the present invention in the lymph nodes or brain.

Determination of the attractive magnetic forces in the preferred embodiment that is necessary to capture agent-containing magnetic particles was achieved by modeling the force on a single magnetic particle in blow flow due to the magnetic field from a bar of magnetic material as follows.

A rigid sphere transported along in Poiseuille flow through a tube has been shown to be subject to radial forces which tend to carry it to a certain equilibrium position at about 0.6 tube radii from the axis, irrespective of the radial position at which the sphere enters the tube (Segre, G. and Silberberg, A. J. Fluid Mech. 1962 14:136). Further, it has been shown that the trajectories of the particles are portions of one master trajectory, and that the origin of the forces causing the radial displacements is in the inertia of the moving fluid (Segre, G. and Silberberg, A. J. Fluid Mech. 1962 14:136).

Before the effects of multiple particles inside a lumen coated three dimensionally with magnetic segments can be modeled, it must first be ensured that were there only one particle, and one bar of magnetic material, that the magnetic attraction would be strong enough that in fluid flow the particle would be able to overcome the drag resistance and attach to the implant. Accordingly, the magnetic force has been modeled as $F_z$, the force in the z direction on a particle by a bar of magnetic material directly placed along the lumen of the vessel. From basic electromagnetic theory it is known that the force on a magnetizable spherical bead in an external magnetic field is given by Equation 1.

$$\text{Eq. 1.} \quad \vec{F} = \mu_0(\vec{m} \cdot \nabla)\vec{H}$$

$$\text{Eq. 2} \quad \vec{m} = \frac{3\chi}{\chi + 3} V\vec{H}$$

In Equations 1 and 2, $\mu_o$ is the magnetic permeability of free space, H is the total external magnetic field, *m* is the magnetic moment of the particle attracted to the implant, *V* is the volume of the particle, and $\chi$ is the magnetic susceptibility of the particle. As can be seen from Equation 1, the force is directly proportional to both the magnetic moment of the particle and the magnetic field gradient. The trajectory of the particle is computed numerically according to Eq. 3 by modeling the movement of the particle towards the wall in a velocity flow field. A particle is captured if its trajectory terminates on the site of the implant:

$$\text{Eq. 3} \quad \Delta z = \frac{F_z}{6\pi\eta\alpha v_x}\Delta x$$

Using values ranging from extreme to moderate to account for varying inducible magnetic field strength, particle size, and blood flow velocity, simulations have shown that for a variety of different physiologically significant parameters, $F_z$ can indeed overcome the vertical drag resistance. These equations must then be modeled based around a circumference equally spaced with magnetic segments, and a fluid containing multiple magnetic particles.

*In vitro* experiments of various kinds have been conducted which demonstrate the utility of devices of the present invention in drug delivery. In one set of experiments, a 316L stainless steel stent was coated with cobalt by electro-deposition. This mesh was then placed within a flow chamber, and magnetic particles were flown through the chamber at varying velocities to assess the capability of the mesh to attract them. Aggregation of magnetic particles on the magnetic stent was increased as compared to a non-magnetic stent.

In another set of experiments, magnetizable beads were embedded in microchannels and magnetic microspheres were injected into the channel under applied magnetic field in order to determine if the embedded beads could capture the microspheres. Again the magnetic microspheres aggregated on the magnetized embedded beads. Experiments using microfabricated fluidic channels were carried out to show a uniqueness of using the magnetizable stent with the surface-modified nanoperticles. Microfluidic channels of various sizes (50x50, 100x100; 500x500 μm cross-section) were fabricated in polydimethyl siloxane (PDMS). The walls of the micro-channels were seeded with superparamagnetic/latex composite microparticles (5μm diameter) (See Forbes ZG, Yellen BB, Barbee KA, Friedman G, An Approach to Targeted Drug Delivery Based on Uniform Magnetic Fields, IEEE Transactions on Magnetics, 39 (5): 3372-3377, (2003)). Top views of the micro-channels and of the entire experimental set-up are schematically illustrated in Figure 2. Various concentration solutions of commercially available magnetic particles of various sizes (1 μm, 3 μm, 5 μm, 7 μm) were observed to flow in the channels while applying external relatively uniform magnetic field varied up to 300 Oe (0.03 Tesla). When no magnetic field is applied, magnetic particle solution flows freely. When the applied magnetic field exceeded 30 Oe aggregation of particles is observed. Stronger fields lead to faster and more pronounced attraction of magnetic particles in solution toward the walls. In lower concentration particle solution, particle chains attracted to the walls form relatively slowly (10s of seconds). In higher concentration particle solution, aggregation of particles on the micro-channel walls occurs very quickly. Examples of aggregation of magnetic carriers in the micro-channels are shown under various flow conditions and fields were observed.

The possibility of making stents that could attract magnetic particle carriers under similar conditions was also confirmed using the technique. Commercially available (e.g., Cordis Corp) stainless steel stents were first confirmed not to attract commercially available magnetic particles (100 nm, 1 μm and 5 μm diameter). A basic set-up for electroplating these stents was developed and stents were coated with 1 μm thick layer of cobalt. The cobalt coated stents were placed into the magnetic particle solution and strongly attracted magnetic particles when a permanent magnet applying a relatively uniform field was placed above the stents. The cobalt coated stents remained covered by the magnetic particles even after washing.

Explanation of Basic Principles of the Proposed Method

The magnetic force dragging isolated magnetic carriers toward the stent is:

$$\vec{F} = \left(\vec{M} \cdot \nabla\right)\vec{B} \qquad (1)$$

where the magnetic moment $\vec{M} \approx \lambda\vec{B}$ of the drug carrying particle is approximately proportional to the total field $\vec{B}$ up until saturation which occurs for most nanoparticles in the range of about 600 Oe (0.06 Tesla). The total field $B = \vec{B}_{ext} + \vec{B}_{stent}$ experienced by the magnetic carriers consists of contributions due to the stent and due to the external magnet. However, while the field of the external magnet is much larger than the field of the stent, it is largely uniform ($\nabla\vec{B}_{ext} \approx 0$). The stent, on the other hand, produces very large gradients near itself because of the presence of very small magnetized features on it. Thus, from (1) and from above arguments, the force on magnetic drug carriers can be approximately written as

$$\vec{F} \approx \lambda\left(\vec{B}_{ext} \cdot \nabla\right)\vec{B}_{stent} \qquad (2)$$

The above formula makes it clear that forces capturing drug carriers are maximized when a strong external uniform magnetic field is superimposed on a field produced by an insert with tiny features maximizing field gradients. This <u>cannot</u> be achieved by a single external magnet. Numerical simulations have been carried out to study efficiency of magnetic carrier capture by magnetized inserts. These simulations have indicated that capture of magnetic carriers as small as 100 nm is feasible with stents that are patterned with magnetized features that are 2-3 μm in diameter and about 200-500 nm in thickness.

It was observed that magnetic carriers may not always be uniformly attracted to stents that are simply coated with magnetic material. This is due to the fact that magnetic domains in such coatings are hard to control. This invention provides several means of patterning the magnetic coating to control domain patterns.

One involve laser assisted electrodeposition of alloys of magnetic metals such as Co, Ni and Fe. Stents will be used as cathodes during the deposition and voltage slightly below electroplating threshold will be applied. Magnetic material can then be electroplated only in those spots that are exposed to a focused laser beam.

Another approach to stent patterning will involve the use of magnetic nanoparticles and nanorods separately prepared. These may either be purchased or made by electroplating into nanotemplates. Magnetic nanoparticles (e.g., ferromagnetic) will then be deposited onto the stents through a process called dielectrophoresis. In this process the

stent will be placed into an aqueous solution containing magnetic nanoparticles in between two insulated electrodes. Application of a relatively high frequency (100 KHz-1MHz) electric field will create strong high frequency electric field gradients on the stent that will attract the nanoparticles.

Another approach will involve recording of magnetic domain pattern on stents using methods closely related to those that are employed in magnetic information storage devices. One such approach will involve laser assisted thermomagnetic recording. In this method the stent is first uniformly magnetized by a strong external field. Subsequently selected spots are heated by a laser in the presence of a reversed magnetic field. The strength of the reversed field is sufficient to reverse magnetization of heated spots, but insufficient to reverse magnetization of unheated spots. In the end, spots that have been heated by a laser, will be magnetized in opposition to the rest of the stent magnetization.

Polylactide-based nanoparticles (NP), sized about 160nm or about 360nm with narrow size distribution formed by a modified emulsification-solvent diffusion or emulsification-solvent evaporation methods, respectively. The NP surface modification with an anionic thiol-reactive derivative of polyallylamine is accomplished photochemically by a brief exposure to long-wave UV light. This permits attachment of affinity adaptors for binding of gene vectors to the surface of these particles. After separation from unreacted polymer, NP can be bioreversibly coated with, for example, a thiolated form of the D1 domain of the Coxsackie-adenovirus (AdV) receptor, to permit delivery of adenoviral gene vectors. Magnetic NP (for use in either gene or drug delivery) are formed by inclusion of iron oxide (preferred from a biocompatibility stand point) nanocrystals in the polymeric matrix. The particle core and the virus were stained with green BODIPY and red Cy3 dyes, respectively, for fluorimetric studies. In addition to binding to the nanoparticle's outer surface via polymeric modification, the therapeutic substance (e.g., a biomolecule) can be incorporated into the particle's core. AdV-NP binding efficacy can be determined by measuring the residual fluorescence of unbound virus following magnetic separation of AdV-NP composite.

Cell culture experiments demonstrating magnetic targeting of cells and loading of these magnetic nanoparticle containing cells onto a magnetized surface were performed. In these studies fluorescent labeled (polylactic polymer was covalently modified with BODIPY 564/570 and used for labeling)

Magnetic nanoparticles comprising the water-soluble polymer as described below (PAA-BzPh or PDT-BzPh) were loaded into cells (arterial smooth muscle cells (A10) in culture) under the influence of a magnetic field. These cells were then grown to confluence, trypsinized and suspended. The suspension was then placed in contact with a cobalt magnetized stainless steel mesh prepared as described above, which demonstrated adhesion of the cells to the magnetized tip of the mesh thereby providing proof of concept for magnetic mediated nanoparticle driven cell delivery. Bright field channel Light Microscopic image of magnetic nanoparticle loaded cells was observed as well as a fluorescent image. In was observed that magnetic nanoprticles were distributed in cells.

The invention was also driven by the desire to develop particles capable of carrying and delivering biomaterial. In certain embodiments, the particles are capable of targeted delivery of biomaterial being guided by a magnetic force, wherein the particles further comprise a coated magnetic field-responsive agent.

Accordingly, this application discloses a particle comprising a matrix-forming agent and a polyelectrolyte-amphiphilic agent adduct, wherein the polyelectrolyte-amphiphilic agent adduct is in physical communication with the matrix-forming agent. In certain embodiments, the particle further comprises a biomaterial in communication with at least one of the polyelectrolyte-amphiphilic agent adduct or the matrix-forming agent.

Advantageously, the particle possesses two compartments for incorporation of a biomaterial, the polyelectrolyte and the matrix-forming agent, wherein one of the compartments or both can be utilized. These particles provide an improved loading of a biomaterial such as, for example, a plasmid DNA by utilizing an ionic binding with a polyelectrolyte (e.g., poly(ethyleneimine) (PEI)) complexed with or adducted to an amphiphilic agent to form a polyelectrolyte-amphiphilic agent adduct. The polyelectrolyte-amphiphilic agent adduct has a strong association with the surface of particles due to its possessing a lipophilic and hydrophilic domains with high affinities to respective media used in the emulsification step of the particle preparation. The polyelectrolyte useful in this invention can be cationic (e.g., PEI) or anionic (e.g., dextran sulfate).

A non-limiting example of the particle comprising an anionic polyelectrolyte is PLA (the matrix-forming polymer)/stearylamine (the amphiphilic agent)/dextran sulfate (polyanion)/PDGF (cationic biomaterial). A non-limiting example of the particle comprising a cationic polyelectrolyte is PLA/oleic acid (the amphiphilic agent)/PEI (the amphiphilic agent)/DNA (anionic biomaterial).

In addition, the biomaterial can be incorporated in these particles by being adsorbed, entangled with or entrapped by the matrix-forming polymer.

Further, these particles may possess magnetic targeting capability and thus make possible the enhanced targeted delivery of the biomaterial to specific cells or organs either in cell culture or *in vivo*, wherein a magnetic field gradient is induced by a magnetic field. These particles interface well with, for example, a nanoscale controllable magnetic patterned device surface (the magnetic device) for binding, release, and repeated loading of drug/gene delivery systems. Thus, in certain embodiments, the particle further comprises a coated magnetic field-responsive agent having a magnetic field-responsive agent in communication with an amphiphilic agent, wherein the coated magnetic field-responsive agent

is in communication with the matrix-forming agent. In certain embodiments, such particle further comprises a biomaterial in communication with at least one of the polyelectrolyte-amphiphilic agent adduct or the matrix-forming agent.

In certain embodiments, this particle comprises a matrix-forming polymer, a polyelectrolyte-amphiphilic agent adduct comprising a first $C_{12}$-$C_{24}$ carboxylate group in physical communication with the matrix-forming polymer, a second amphiphilic agent comprising a second $C_{12}$-$C_{24}$ carboxylate group in communication with the matrix-forming polymer; and a magnetic-field responsive agent in communication with a second $C_{12}$-$C_{24}$ carboxylate group. In certain embodiments, the particle further comprises a stabilizer. In certain embodiments, the particle further comprises a biomaterial in communication with the polyelectrolyte-amphiphilic agent adduct and optionally with the matrix-forming polymer.

In a preferred embodiment, the particle comprises poly(lactic acid) (PLA), PEI-oleic acid adduct, Pluronic F-68, magnetite coated with oleic acid and DNA, wherein PLA, PEI-oleic acid adduct, and Pluronic F-68 were premixed, mixed with a dispersion of magnetite/oleic acid in organic solvent, and emulsified in water, followed by removal of organic solvent.

Further, the particle can be a magnetic particle comprising a matrix-forming polymer; and a coated magnetic field-responsive agent comprising a magnetic field-responsive agent and a second amphiphilic agent, wherein the coated magnetic field-responsive agent is in communication with the matrix-forming polymer, provided that the magnetic particle is free of a polyelectrolyte. A used herein, the term "magnetic particle" denotes a particle possessing magnetic capabilities conferred by the coated magnetic field-responsive agent but in the absence of the polyelectrolyte to differentiate from a particle possessing magnetic capabilities and comprising the polyelectrolyte. In certain embodiments, the magnetic particle further comprises a biomaterial in communication with the matrix-forming polymer.

The particle can be used for delivery of biomaterial entrapped in or adsorbed on the matrix-forming polymer and/or associated with the particle surface via the polyelectrolyte for various applications such as, for example, drug therapy, chemotherapy, chemoembolization, hyperthermic cancer treatment, diagnostics, and radiotherapy.

One of the applications for this formulation is a targeted gene or drug delivery to the heart. In one embodiment of this application, a cardiac catheterization is carried out with positioning of a powerful magnet in the part of the heart where gene delivery is desired, for example, in the right atrium (a targeted tissue). Next, the particles described above (e.g., the biodegradable PEI-modified magnetic nanoparticles loaded with DNA) are administered to the coronary artery (e.g., by injection) and the myocardial circulation together with the magnetic field localizes the bulk of the particles in the desired area, the right atrial myocardium. A similar approach could be applied to virtually any target cell or organ region by providing the particles of the invention and a magnetic device associated with the targeted cell or the targeted tissue.

The above particles can be used also for the prevention, diagnostic, or treatment of various conditions or disorders such as, for example, tumors, gastro-intestinal disease, pulmonary and bronchial disorders by delivering the appropriate biomaterial. These particles can also be used in hormonal therapy and anesthetic medication.

Also, the above particles can be used as an analytical tool, for example for screening.

Delivery of biomaterial under influence of magnetic force includes reversible movement of particles from one target site to another.

In certain embodiments, the particle further comprises a biomaterial in communication with at least one of the polyelectrolyte-amphiphilic agent adduct or the matrix-forming agent, wherein the particle is free of the magnetic field-responsive agent.

The particle, its components, and methods of making the particle will be described in detail below.

PARTICLE

The term "particle" as used herein denotes a solid colloidal particle having a diameter of about 5 nm to about 10 microns. The particle can be a sphere or a capsule, wherein the sphere is composed of a solid matrix, while the capsule has an oil-based or water-based core surrounded by the matrix-forming polymer. An example of a magnetic particle is shown in Fig. 9. The particle can have one or more coated magnetic field-responsive agents incorporated within the matrix-forming polymer. Preferably, multiple coated magnetic nanocrystals are incorporated in the core of the particle.

MATRIX-FORMING AGENT

The matrix-forming agent used in the invention includes synthetic or natural polymers and non-polymeric substances.

The term "matrix-forming polymer" as used herein denotes a synthetic or a natural polymer, which forms the core of the particle. The matrix-forming polymer can be biodegradable, non-biodegradable, biocompatible, and is water-insoluble. The release kinetics of biomaterial from nanoparticles can be determined by the rate of the matrix-forming polymer's degradation, diffusion or dissolution of material entrapped in the particle or desorption of surface-bound

substances.

Non-limiting examples of natural polymers are proteins, polysaccharides and lipids as described by Quintanar-Guerrero et al., supra and Kumar, supra. Non-limiting examples of synthetic polymers are poly(ester)s, poly(urethane)s, poly(alkylcyanoacrylate)s, poly(anhydride)s, poly(ethylenevinyl acetate), poly(lactone)s, poly(styrene)s, poly(amide)s, poly(acrylonitrile)s, poly(acrylate)s, poly(metacrylate)s, poly(orthoester)s, poly(ether-ester)s, poly(tetrafluoroethylene)s, mixtures thereof and copolymers thereof. In certain embodiments, the poly(ester) is a member selected from the group consisting of poly(lactide), poly(glycolide), poly(lactide-co-glycolide), poly(ε-caprolactone), poly(dioxanone), poly(hydroxybutyrate), and poly(ethylene terephthalate).

Non-limiting examples of non-polymeric substances are solid lipids such as glycerides and fatty acids.

AMPHIPHILIC AGENT

The term "amphiphilic agent" as used herein denotes a biocompatible agent having a molecule comprising a reactive group and a lipophilic group. Non-limiting examples of amphiphilic agents are fatty acids and lipids as well as salts thereof such as carboxylates, phosphonates, bisphosphonates, phosphates, sulfonates, and sulfates. In certain embodiments, fatty acids are $C_{12}$-$C_{24}$ carboxylic acids and salts or esters thereof; the preferred fatty acid is oleic acid. In certain embodiments, lipids are phospholipids such as phosphatidylglycerol and phosphatidylinositol. Amphiphilic agent can also be cationic, such as stearylamine, 3β-(N-[dimethylamine ethane] carbamoyl) cholesterol (DC-Chol), N-[1-(2,3-dioleoyloxy)propyl]-N,N,N-trimethylammonium chloride (DOTAP).

One of the functions of the amphiphilic agent is to provide a connection between the matrix-forming agent and the polyelectrolyte.

The reactive group of the amphiphilic agent is a polar chemical group such as, for example, a carboxylate group, aphosphonate group, a bisphosphonate group, a phosphate group, a sulfonate group, and a sulfate group. The association between the amphiphilic agent and the polyelectrolyte may be ionic (e.g., an ammonium carboxylate ion-pair) or covalent (e.g. via amide, imine, urethane, urea, alkyl bond, etc.).

The lipophilic group of the amphiphilic agent is a hydrocarbon chain with low water affinity, preferably a $C_4$-$C_{24}$ chain having all saturated bonds (e.g., lauric acid and palmitic acid) or at least one unsaturated bond (e.g., oleic acid). The hydrocarbon chain can present a cyclic structure (e.g. cholesterol or cholic acid). The hydrocarbon chain can further have one or more hydrogen atoms substituted with for example an aromatic group. The hydrocarbon chain can further have one of more carbon atoms substituted with heteroatoms (e.g., nitrogen, sulfur, and oxygen).

The adduct of the amphiphilic agent and the polyelectrolyte can be preformed or made *in situ* in the course of the particle preparation.

POLYELECTROLYTE

The polyelectrolyte used in the invention provides a connection between the matrix-forming polymer and the biomaterial, wherein the polyelectrolyte is associated with the matrix-forming polymer through the amphiphilic agent. The polyelectrolyte can be cationic or anionic.

In one embodiment, the polyelectrolyte and the biomaterial are ionically associated. For example, the polyelectrolyte is cationic (e.g., poly(ethyleneimine)) and the biomaterial is anionic (e.g., DNA) or the polyelectrolyte is anionic (e.g., dextran sulfate) and the biomaterial is cationic (e.g., PDGF).

Association between the biomaterial and the polyelectrolyte-amphiphilic agent adduct can also be covalent, wherein reactive groups of the biomaterial chemically react with reactive groups of the polyelectrolyte-amphiphilic agent.

In certain embodiments, the polyelectrolyte is a member selected from the group consisting of poly(ethyleneimine), poly(propyleneimine), poly[N-ethyl-4-vinyl pyridinium bromide], polyamidoamine dendrimer, poly(allylamine) and derivatives thereof.

Preferably, PEI used in the invention has a molecular weight of about 25 KDa, and more preferably about 14 KDa or less to enhance its elimination from the body or is a biodegradable derivative thereof.

PEI with the molecular weight of at least 25 KDa is known to be toxic to cells while decreasing the size of PEI reduces the toxicity but it also reduces the efficacy of gene transfer. (See Forrest et al., Gosselin et al., ).

Examples of biodegradable PEI derivatives useful in the invention can be found in Gosselin et al., Efficient gene transfer using reversibly cross-linked low molecular weight polyethylenimine, Bioconjug Chem. 2001 Nov-Dec;12(6):989-94, and expected to result in further reduction of toxic effects. PEI derivatives can be prepared by cross-linking 800 Da PEI with dithiobis(succinimidylpropionate) (DSP) and/or dimethyl 3,3'-dithiobispropionimidate 2HCl (DTBP).

Forrest et al., A degradable polyethylenimine derivative with low toxicity for highly efficient gene delivery, Bioconjug Chem. 2003 Sep-Oct; 14(5):934-40); disclose highly branched 14-30 KDa polycations that are biodegradable analogs of 25 KDa PEI produced by addition of amino groups on 800 Da PEI to diacrylates such as, for example, 1,3-butanediol diacrylate of varying spacer length. These or similar derivatives can also be useful in the invention.

MAGNETIC FIELD-RESPONSIVE AGENT

A magnetic field-responsive agent as used herein is a paramagnetic, superparamagnetic, or ferromagnetic substance capable of moving under influence of a magnetic force. In certain embodiments, the magnetic field-responsive agent is a member selected from the group consisting of iron, cobalt or nickel, alloys thereof, oxides thereof and mixed oxides/hydroxides of Fe(II) and/or Fe(III) with at least one of Co(II), Mn(II), Cu(II), Ni(II), Cr(III), Gd(III), Dy(III), and Sm(III). Preferably, the magnetic field-responsive agent is at least one of $Fe_3O_4$, gamma-$Fe_2O_3$, or a mixture thereof. Preferably, the magnetic field-responsive agent is iron oxide in a shape of nanocrystals.

The magnetic field-responsive agent can be prepared by methods known in the art in various shapes and sizes (see Hyeon T., Chemical Synthesis of Magnetic Nanoparticles. The Royal Society of Chemistry 2003, Chem. Commun., 2003, 927-934). In certain embodiments, iron oxide nanocrystals were obtained by precipitation of mixed iron chlorides in the presence of a base in aqueous medium (see Khalafalla SE. Magnetic fluids, Chemtech 1975, Sept.: 540-547).

The term "coated magnetic field-responsive agent" as used herein denotes a magnetic field-responsive agent in communication with an amphiphilic agent (an amphiphilic agent can be defined as "first" or "second" to indicate that these agents can be different substances, however embodiments in which these agents are the same are contemplated as well). In the particle of the invention, the coated magnetic field-responsive agent is in communication with the matrix-forming agent.

STABILIZING AGENT

Optionally, the particle includes a stabilizing agent. Non-limiting examples of stabilizing agents include poly(sorbate) (e.g., TWEEN (Merck and Co. Inc., Whitehouse Station New Jersey, USA)), sorbitan ester, ethylene oxide-propylene oxide block copolymers (e.g., poloxamer), poloxamine, poly(ethylene glycol) (PEG), alkyl polyethylene glycol ether, PEG-based non-ionic surfactants (e.g., fatty acid polyethylene glycol ester and mixtures thereof). Examples of the functions of such stabilizing agents include providing steric protection, contributing to the stability in high ionic strength media in vitro and in serum in vivo, preventing rapid sequestration of the nanoparticles by the reticuloendothelial system following an IV injection.

BIOMATERIAL

The biomaterial usable in the present invention can be any molecule or macromolecule having a therapeutical utility. In certain embodiments of the composition, the biomaterial is a member selected from the group consisting of a nucleic acid, a protein, a peptide, an oligonucleotide, an antibody, an antigen, a viral vector, a bioactive polypeptide, a polynucleotide coding for the bioactive polypeptide, a cell regulatory small molecule, a gene therapy agent, a gene transfection vector, a receptor, a cell, a drug, a drug delivering agent, an antimicrobial agent, an antibiotic, an antimitotic, an antisecretory agent, an anti-cancer chemotherapeutic agent, steroidal and non-steroidal anti-inflammatories, a hormone, a proteoglycan, a glycosaminoglycan, a free radical scavenger, an iron chelator, an antioxidant, an imaging agent, and a radiotherapeutic agent.

In certain embodiments of the composition, the biomaterial is any molecule or macromolecule to which a suitable ionizable reactive group, such as, for example, a carboxy (-COOH) group or an amino ($-NH_2$) group is attached.

The biomaterial can associate with the particle in several ways. In one embodiment, the biomaterial is in ionic association with the charged groups of the polyelectrolyte-amphiphilic agent adduct (e.g., the carboxylate group) while the lipophilic part of the polyelectrolyte-amphiphilic agent adduct is in association (i.e., anchored in the matrix or chemically bound to it) with the matrix-forming polymer of the particle. Association between the biomaterial and the polyelectrolyte-amphiphilic agent adduct can also be covalent, wherein reactive groups of the biomaterial chemically react with reactive groups of the polyelectrolyte-amphiphilic agent.

In certain embodiments, the biomaterial can be in physical association (i.e., entrapped or adsorbed) with the matrix-forming polymer.

In certain embodiments, physical interactions as well as ionic and/or covalent and are utilized.

Suitable biomaterial include pharmaceuticals, nucleic acid sequences, such as transposons, signaling proteins that facilitate wound healing, such as TGF-β, FGF, PDGF, IGF and GH proteins that regulate cell survival and apoptosis, such as Bcl-1 family members and caspases; tumor suppressor proteins, such as the retinoblastoma, p53, PAC, DCC. NF1, NF2, RET, VHL and WT-1 gene products; extracellular matrix proteins, such as laminins, fibronectins and integrins; cell adhesion molecules such as cadherins, N-CAMs, selectins and immunoglobulins; anti-inflammatory proteins such as Thymosin beta-4, IL-10 and IL-12.

In certain embodiments, the biomaterial includes at least one of: heparin, covalent heparin, or another thrombin

inhibitor, hirudin, hirulog, argatroban, D-phenylalanyl-L-poly-L-arginyl chloromethyl ketone, or another antithrombogenic agent, or mixtures thereof; urokinase, streptokinase, a tissue plasminogen activator, or another thrombolytic agent, or mixtures thereof; a fibrinolytic agent; a vasospasm inhibitor; a calcium channel blocker, a nitrate, nitric oxide, a nitric oxide promoter or another vasodilator; an antimicrobial agent or antibiotic; aspirin, ticlopidine, a glycoprotein IIb/IIIa inhibitor or another inhibitor of surface glycoprotein receptors, or another antiplatelet agent; colchicine or another antimitotic, or another microtubule inhibitor, dimethyl sulfoxide (DMSO), a retinoid or another antisecretory agent; cytochalasin or another actin inhibitor; a remodeling inhibitor; deoxyribonucleic acid, an antisense nucleotide or another agent for molecular genetic intervention; methotrexate or another antimetabolite or antiproliferative agent; tamoxifen citrate, Taxol™ or derivatives thereof, or other anti-cancer chemotherapeutic agents; dexamethasone, dexamethasone sodium phosphate, dexamethasone acetate or another dexamethasone derivative, or another anti-inflammatory steroid or a non-steroidal anti-inflammatory agent; cyclosporin or another immunosuppressive agent; trapidal (a PDGF antagonist), angiogenin, angiopeptin (a growth hormone antagonist), a growth factor or an anti-growth factor antibody, or another growth factor antagonist; dopamine, bromocriptine mesylate, pergolide mesylate or another dopamine agonist; radiotherapeutic agent; iodine-containing compounds, barium-containing compounds, gold, tantalum, platinum, tungsten or another heavy metal functioning as a radiopaque agent; a cellular component; captopril, enalapril or another angiotensin converting enzyme (ACE) inhibitor; ascorbic acid, alpha tocopherol, superoxide dismutase, deferoxamine, a 21-amino steroid (lasaroid) or another free radical scavenger, iron chelator or antioxidant; a $^{14}C$-, $^{3}H$-, $^{32}P$- or $^{36}S$-radiolabelled form or other radiolabelled form of any of the foregoing; a hormone; estrogen or another sex hormone; AZT or other antipolymerases; acyclovir, famciclovir, rimantadine hydrochloride, ganciclovir sodium or other antiviral agents; 5-aminolevulinic acid, meta-tetrahydroxyphenylchlorin, hexadecafluoro zinc phthalocyanine, tetramethyl hematoporphyrin, rhodamine 123 or other photodynamic therapy agents; an IgG2 Kappa antibody against Pseudomonas aeruginosa exotoxin A and reactive with A431 epidermoid carcinoma cells, monoclonal antibody against the noradrenergic enzyme dopamine beta-hydroxylase conjugated to saporin or other antibody targeted therapy agents; gene therapy agents; and enalapril and other prodrugs, or a mixture of any of these.

Additionally, the biomaterial can be a component of any affinity-ligand pair. Examples of such affinity ligand pairs include avidin-biotin and IgG-protein A. Furthermore, the biomaterial can be either component of any receptor-ligand pair. One example is transferrin and its receptor. Other affinity ligand pairs include powerful hydrogen bonding or ionic bonding entities such as chemical complexes. Examples of the latter include metallo-amine complexes. Other such attractive complexes include nucleic acid base pairs, via immobilizing oligonucleotides of a specific sequence, especially antisense. Nucleic acid decoys or synthetic analogues can also be used as pairing agents to bind a designed gene vector with attractive sites. Furthermore, DNA binding proteins can also be considered as specific affinity agents; these include such entities as histones, transcription factors, and receptors such as the gluco-corticoid receptor.

In one preferred embodiment, the biomaterial is an anti-nucleic acid antibody. The antibody can therefore specifically bind a nucleic acid, which encodes a product (or the precursor of a product) that decreases cell proliferation or induces cell death, thereby mitigating the problem of restenosis in arteries and other vessels. The nucleic acid that is tethered to a support via the antibody can efficiently transfect/transducer cells. In general terms, the field of "gene therapy" involves delivering into target cells some polynucleotide, such as an antisense DNA or RNA, a ribozyme, a viral fragment, or a functionally active gene, that has a therapeutic or prophylactic effect on the cell or the organism containing it. The antibody of the composition can be a full-length (i.e., naturally occurring or formed by normal immunoglobulin gene fragment recombinatorial processes) immunoglobulin molecule (e.g., an IgG antibody, or IgM or any antibody subtype) or an immunologically active (i.e., specifically binding) portion of an immunoglobulin molecule. The antibody comprises one or more sites, which specifically bind with a nucleic acid (i.e., which does not substantially bind other types of molecules). The binding site can be one that binds specifically with a nucleic acid of a desired type without regard to the nucleotide sequence of the nucleic acid. The binding site can, alternatively, be one which binds specifically only with a nucleic acid comprising a desired nucleotide sequence.

The complex formed between a polynucleotide and a cognate antibody can be immobilized on a variety of surfaces such that, when the surface is exposed to a physiological environment *in situ,* the attached polynucleotide is released, over time, in a manner that enhances delivery of the polynucleotide to cells in the proximity. DNA transfer by way of immunospecific tethering has previously been shown to maintain the nucleic acid in regions that are subject to gene therapy.

Examples of suitable antibodies include Fv, F(ab), and $F(ab')_2$ fragments, which can be generated is conventional fashion, as by treating an antibody with pepsin or another proteolytic enzyme. The nucleic acid-binding antibody useful in the present invention can be polyclonal antibody or a monoclonal antibody. A "monoclonal" antibody comprises only one type of antigen binding site that specifically binds with the nucleic acid. A "polyclonal" antibody can comprise multiple antigen binding sites that specifically bind the nucleic acid. An antibody employed in this invention preferably is a full-length antibody or a fragment of an antibody, such as $F(ab')_2$, that possesses the desired binding properties.

A nucleic acid for use in the present invention can be any polynucleotide that one desires to transport to the interior of a cell. In this context, a "therapeutic polynucleotide" is a polymer of nucleotides that, when provided to or

expressed in a cell, alleviates, inhibits, or prevents a disease or adverse condition, such as inflammation and/or promotes tissue healing and repair (*e.g.,* wound healing). The nucleic acid can be composed of deoxyribonucleosides or ribonucleosides, and can have phosphodiester linkages or modified linkages, such as those described below. The phrase "nucleic acid" also encompasses polynucleotides composed of bases other than the five that are typical of biological systems: adenine, guanine, thymine, cytosine and uracil.

A suitable nucleic acid can be DNA or RNA, linear or circular and can be single-or-double-stranded. The "DNA" category in this regard includes: cDNA; genomic DNA; triple helical, supercoiled, Z-DNA and other unusual forms of DNA; polynucleotide analogs; an expression construct that comprises a DNA segment coding for a protein, including a therapeutic protein; so-called "antisense" constructs that, upon transcription, yield a ribozyme or an antisense RNA; viral genome fragments, such as viral DNA; plasmids and cosmids; and a gene or gene fragment.

The nucleic acid also can be RNA, for example, antisense RNA, catalytic RNA, catalytic RNA/protein complex (*i.e*., a "ribozyme"), and expression construct comprised of RNA that can be translated directly, generating a protein, or that can be reverse transcribed and either transcribed or transcribed and then translated, generating an RNA or protein product, respectively; transcribable constructs comprising RNA that embodies the promoter/regulatory sequence(s) necessary for the generation of DNA by reverse transcription; viral RNA; and RNA that codes for a therapeutic protein, *inter alia.* A suitable nucleic acid can be selected on the basis of a known, anticipated, or expected biological activity that the nucleic acid will exhibit upon delivery to the interior of a target cell or its nucleus.

The length of the nucleic acid is not critical to the invention. The nucleic acid can be linear or circular double-stranded DNA molecule having a length from about 100 to 10,000 base pairs in length, although both longer and shorter nucleic acids can be used.

The nucleic acid can be a therapeutic agent, such as an antisense DNA molecule that inhibits mRNA translation. Alternatively, the nucleic acid can encode a therapeutic agent, such as a transcription or translation product which, when expressed by a target cell to which the nucleic acid-containing composition is delivered, has a therapeutic effect on the cell or on a host organism that includes the cell. Examples of therapeutic transcription products include proteins (e.g., antibodies, enzymes, receptors-binding ligands, wound-healing proteins, anti-restenotic proteins, anti-oncogenic proteins, and transcriptional or translational regulatory proteins), antisense RNA molecules, ribozymes, viral genome fragments, and the like. The nucleic acid likewise can encode a product that functions as a marker for cells that have been transformed, using the composition. Illustrative markers include proteins that have identifiable spectroscopic properties, such as green fluorescent protein (GFP) and proteins that are expressed on cell surfaces (*i.e.,* can be detected by contacting the target cell with an agent which specifically binds the protein). Also, the nucleic acid can be a prophylactic agent useful in the prevention of disease.

A nucleic-acid category useful in the present invention encompasses polynucleotides that encode proteins that affect wound-healing. For example, the genes *egf, tgf, kgf, hb-egf, pdgf, igf, fgf-1, fgf-2, vegf,* other growth factors and their receptors, play a considerable role in wound repair.

Another category of polynucleotides, coding for factors that modulate or counteract inflammatory processes, also is useful for the present invention. Also relevant are genes that encode an anti-inflammatory agent such as MSH, a cytokine such as IL-10, or a receptor antagonist that diminishes the inflammatory response.

Suitable polynucleotides can code for an expression product that induces cell death or, alternatively, promotes cell survival, depending on the nucleic acid. These polynucleotides are useful not only for treating tumorigenic and other abnormal cells but also for inducing apoptosis in normal cells. Accordingly, another notable nucleic-acid category for the present invention relates to polynucleotides that, upon expression, encode an anti-oncogenic protein or, upon transcription, yield an anti-oncogenic antisense oligonucleotide. In this context, the phrases "anti-oncogenic protein" and "anti-oncogenic antisense oligonucleotide" respectively denote a protein or an antisense oligonucleotide that, when provided to any region where cell death is desired, or the site of a cancerous or precancerous lesion in a subject, prevents, inhibits, reverses abnormal and normal cellular growth at the site or induces apoptosis of cells. Delivery of such a polynucleotide to cells, pursuant to the present invention, can inhibit cellular growth, differentiation, or migration to prevent movement or unwanted expansion of tissue at or near the site of transfer. Illustrative of this anti-oncogenic category are polynucleotides that code for one of the known anti-oncogenic proteins. Such a polynucleotide would include, for example, a nucleotide sequence taken or derived from one or more of the following genes: *abl, akt2, apc, bcl2-alpha, bcl2-beta, bcl3, bcl3, bcl-x, bad, bcr, brcal, brca2, cbl, ccndl, cdk4, crk-II, csflr/fms, dbl, dcc, dpc4/smad4, e-cad, e2fl/rbap, egfr/erbb-1, elk1, elk3, eph, erg, ets1, ets2, fer, fgr/src2, fos, fps/fes, fral, fra2, fyn, hck, hek, her2/erbb-2/neu, her3/erbb-3, her4/erbb-4, hras1, hst2, hstfl, ink4a, ink4b, int2/fgf3, jun, junb, jund, kip2, kit, kras2a, kras2b, ck, lyn, mas, max, mcc, met, mlh1, mos, msh2, msh3, msh6, myb, myba, mybb, myc, mycl1, mycn, nfl, nf2, nras, p53, pdgfb, pim1, pms1, pms2, ptc, pten, raft, rb1, rel, ret, ros1, ski, src1, tal1, tgfbr2, thra1, thrb, tiaml, trk, vav, vhl, waf1,wnt1, wnt2, wt1 and yes1*. By the same token, oligonucleotides that inhibit expression of one of these genes can be used as anti-oncogenic antisense oligonucleotides.

Nucleic acids having modified inter-nucleoside linkages also can be used in composition according to the present invention. For example, nucleic acids can be employed that contain modified internucleoside linkages, which

exhibit increased nuclease stability. Such polynuclotides include, for example, those that contain one or more phosphonate, phosphorothioate, phosphorodithioate, phosphoramidate methoxyethyl phosphoramidate, formacetal, thioformacetal, diisopropylsilyl, acetamidate, carbamate, dimethylene-sulfide ($-CH_2-S-CH_2-$), dimethylene-sulfoxide($-CH_2-SO-CH_2-$), dimethylenesulfone ($-CH_2-SO_2-CH_2-$), 2'-O-alkyl, and 2'-deoxy-2'-fluoro-phosphorothioate internucleoside linkages.

For present purposes, a nucleic acid can be prepared or isolated by any conventional means typically used to prepare or isolate nucleic acids. For example, DNA and RNA can be chemically synthesized using commercially available reagents and synthesizers by known methods. RNA molecules also can be produced in high yield via *in vitro* transcription techniques, using plasmids such as SP65, available from Promega Corporation (Madison, WI). The nucleic acid can be purified by any suitable means. For example, the nucleic acid can be purified by reverse-phase or ion exchange HPLC, size exclusion chromatography, or gel electrophoresis. Of course, the skilled artisan will recognize that the method of purification will depend in part on the size of the DNA to be purified. The nucleic acid also can be prepared via any of the innumerable recombinant techniques that are known or that are developed hereafter.

A suitable nucleic acid can be engineered into a variety of known host vector systems that provide for replication of the nucleic acid on a scale suitable for the preparation of an inventive composition.

Vector systems useful in the present invention can be viral or non-viral. Particular examples of viral vector systems include adenovirus, retrovirus, adeno-associated virus and herpes simplex virus. Preferably, an adenovirus vector is used. A non-viral vector system includes a plasmid, a circular, double-stranded DNA molecule. Viral and nonviral vector systems can be designed, using known methods, to contain the elements necessary for directing transcription, translation, or both, of the nucleic acid in a cell to which is delivered. Methods known to the skilled artisan can be used to construct expression constructs having the protein coding sequence operably linked with appropriate transcriptional/translational control signals. These methods include *in vitro* recombinant DNA techniques and synthetic techniques. For instance, see Sambrook et al., 1989, MOLECULAR CLONING: A LABORATORY MANUAL (Cold Spring Harbor Laboratory, New York).

A nucleic acid encoding one or more proteins of interest can be operatively associated with a variety of different promoter/regulator sequences. The promoter/regulator sequences can include a constitutive or inducible promoter, and can be used under the appropriate conditions to direct high level or regulated expression of the gene of interest. Particular examples of promoter/regulatory regions that can be used include the cytomegalovirus (CMV) promoter/regulatory region and the promoter/regulatory regions associated with the SV40 early genes or the SV40 late genes.

It also is within the scope of the present invention that the employed nucleic acid contains a plurality of protein-coding regions, combined on a single genetic construct under control of one or more promoters. The two or more protein-coding regions can be under the transcriptional control of a single promoter, and the transcript of the nucleic acid can comprise one or more internal ribosome entry sites interposed between the protein-coding regions. Thus, a myriad of different genes and genetic constructs can be utilized.

## METHODS OF MAKING PARTICLES

Further disclosed is a method of making a particle usable in the invention, the method comprising: (i) providing (a) the matrix-forming agent, (b) a polyelectrolyte, (c) a first amphiphilic agent, (d) a first medium, (e) a second medium, and optionally providing a stabilizer; (ii) mixing at least the matrix-forming agent, the first medium, and the second medium and optionally the polyelectrolyte, the first amphiphilic agent, and/or the stabilizer to give a first mixture; (iii) emulsifying the first mixture to give a first emulsion; and (iv) removing the first medium and thereby forming the particle, on a condition that the polyelectrolyte, the first amphiphilic agent, and the stabilizer are provided to at least one of the first medium, the second medium, the first mixture, the first emulsion, or the particle such that the polyelectrolyte and the first amphiphilic agent form the polyelectrolyte-amphiphilic agent adduct.

In certain embodiments of the method, the polyelectrolyte, the first amphiphilic agent, and optionally the stabilizer are combined with the matrix-forming agent prior to mixing with the first medium or the second medium.

The term "first medium" as used herein denotes a solution, a micellar solution, an emulsion, or a suspension. In certain embodiments of the method, the first medium comprises an organic solvent. In certain embodiments of the method, the first medium comprises the organic solvent selected from the group consisting of chloroform, dichloromethane, tetrahydrofuran, acetone, ethanol, hexane, heptane, methylethylketone, propylene carbonate, ethyl acetate, acetylacetone, acetic anhydride, dimethylsulfoxide, dimethylformamide, acetonitrile and mixtures thereof. In certain embodiments, the organic solvent is provided as a ratio of at least two different organic solvents wherein the ratio influences a size of the particle. In one variant, the least two different organic solvents are tetrahydrofuran and chloroform provided at the ratio of about 0.1 to about 3 and the particle's diameter is about 370 nm to about 100 nm.

Emulsification can be performed by methods known in the art such as ultrasonication, high pressure homogenization, and microfluidization as described by Quintanar-Guerrero et al., supra.

Methods of preparing particles usable in the invention avoid using harsh conditions (e.g. extreme pH, elevated

temperatures), which can cause degradation of the matrix-forming polymer and/or some biomaterials. Room temperature is preferred for steps involving addition of the biomaterial.

Preparations of coated magnetic field-responsive agent can be conducted at elevated temperatures in the absence of biomaterial.

In certain embodiments of the method, the second medium is a member selected from the group consisting of water, alcohols, and liquid hydrocarbons.

In certain embodiments, the method further comprises providing a biomaterial.

In certain embodiments of the method, the biomaterial is provided to at least one of the first mixture, the first emulsion and/or the particle. It may be added as is or in a co-solvent (for example, methotrexate (the biomaterial) can be added in dimethylsulfoxide (the co-solvent) to the first medium. Further, it can be incorporated in either component of the first medium, i.e. as a part of a solution, a micellar solution, an emulsion, or a suspension depending on solubility of the biomaterial in particular substances used.

In another embodiment, the method further comprises providing a coated magnetic field-responsive agent to at least one of the first medium, the second medium, and/or the first mixture. In certain embodiments, the coated magnetic field-responsive agent is provided by combining a magnetic field-responsive agent and a second amphiphilic agent in the presence of the first medium, the second medium, or the first mixture. Preferably, the coated magnetic field-responsive agent is dispersed in the first medium and mixed with the matrix-forming agent, the polyelectrolyte, the first amphiphilic agent, and the stabilizer prior to mixing with the second medium as shown in Fig. 5. In one variant of this embodiment, the method further comprises providing a biomaterial, wherein the biomaterial is provided to at least one of the first mixture, the first emulsion and/or the particle. Preferably, the biomaterial is provided to the particle, in such a manner that the biomaterial is in communication with the polyelectrolyte-first amphiphilic agent adduct, provided that the polyelectrolyte, the first amphiphilic agent, and the stabilizer are combined with the matrix-forming agent before mixing with the first medium.

Further disclosed is a method of making a magnetic particle usable in the invention, the method comprising: (i) providing the matrix-forming polymer, the coated magnetic field-responsive agent, a first medium and a second medium, and optionally providing a stabilizer; (ii) mixing at least the matrix-forming polymer, the first medium, and the second medium to give a second mixture; (iii) emulsifying the second mixture to give a second emulsion; and (iv) removing the first medium and thereby forming the particle, on a condition that the coated magnetic field-responsive agent and optionally the stabilizer are provided to at least one of the first medium, the second medium, or the second mixture. Preferably, the coated magnetic field-responsive agent is provided by combining the magnetic field-responsive agent and the second amphiphilic agent in a presence of the first medium, the second medium, or the second mixture. One variant of this embodiment includes further providing a biomaterial. The biomaterial can be provided to at least one of the second mixture, the second emulsion or the magnetic particle.

METHODS OF DELIVERY OF BIOMATERIAL TO TARGET CELL OR TARGET TISSUE

Also disclosed is a method of delivery of a biomaterial to a target cell or a target tissue, the method comprising: administering a particle comprising the matrix-forming agent, polyelectrolyte-amphiphilic agent adduct, the coated magnetic field-responsive agent and the biomaterial; optionally providing a magnetic device associated with the target cell or the target tissue; applying a magnetic force to the particle; and guiding the particle by the magnetic force and thereby delivering the biomaterial to the target cell or the target tissue.

The source of magnetic field can be any source known in the art, e.g., an electromagnet. Magnetic field can be applied internally by placing a magnetic device inside a body in need of delivery of biomaterial; the magnetic field can be applied externally or as a combination of both.

Further disclosed is a method of delivery of a biomaterial to a target cell or a target tissue, the method comprising: administering a particle comprising the matrix-forming agent, the coated magnetic field-responsive agent, and the biomaterial, wherein the particle is free of the polyelectrolyte; providing a magnetic device associated with the target cell or the target tissue; applying a magnetic force to the particle; and guiding the particle by the magnetic force and thereby delivering the biomaterial to the target cell or the target tissue.

Also disclosed is a method of delivery of a biomaterial to a cell or a tissue, the method comprising: administering a particle comprising the matrix-forming agent, the polyelectrolyte-amphiphilic agent adduct, and the biomaterial, wherein the particle is free of the magnetic field-responsive agent; and delivering the biomaterial to the cell or tissue using the particle as a carrier, wherein the cell is optionally contacted with a transfection agent prior to said delivering.

Administering of the particle can be done, for example, by layering, spraying, pouring, injection, inhalation, or ingestion or a combination of any of the above.

The invention will be illustrated in more detail with reference to the following Examples, but it should be understood that the present invention is not deemed to be limited thereto.

EXAMPLES

EXAMPLE 1

PREPARATION OF A COATED MAGNETIC-FIELD RESPONSIVE AGENT

The formulation of superparamagnetic biodegradable nanoparticles involves two main steps: forming a long-chain carboxylic acid stabilized iron oxide nanocrystals and incorporation of the iron oxide nanocrystals in the biodegradable polymeric matrix of PLA-based nanoparticles formulated with PEI. The first step involves co-precipitation of ferric and ferrous chlorides in the presence of aqueous base solution (NaOH, 0. IN) with subsequent coating with oleic acid, thus rendering the nanocrystal surface lipophilic (see De Cuyper, supra; and Khalafalla supra). The obtained stabilized iron oxide nanocrystals were separated by extraction or centrifugation/magnetic sedimentation with subsequent resuspension in chloroform or a mixture of chlorofom and tetrahydrofuran.

1 ml of an aqueous solution containing 65 mg of $FeCl_3$ hexahydrate, 32 mg of $FeCl_2$ tetrahydrate and 50 mg of Pluronic F-68 (as an optional stabilizer) (BASF Corp.) were rapidly mixed with 10 ml aqueous solution of NaOH (0.1M). Next, 200 mg of oleic acid were added dropwise, and the mixture was degassed in argon. The mixture was heated to 90° C in a water bath for 5 min and cooled to room temperature. The mixture in a form of a suspension was vortexed with 5 ml of $CHCl_3$ to extract thus formed iron oxide nanocrystals, and the bottom layer was further used to prepare nanoparticles.

PREPARATION OF NANOPARTICLES

Biodegradable matrix-forming polymer (polylactide) and PEI were incorporated into the iron oxide dispersion in the organic medium, and the organic phase thus obtained is emulsified in water followed by the organic solvent's evaporation (emulsification-solvent evaporation or the emulsification solvent-diffusion method) and nanoparticles filtration as in the example below.

200 mg of PLA (D,L-PLA (70-120 KDa) Sigma), 50 mg of Pluronic F-68 (BASF Corp.), 100 mg of PEI (25 KDa; Aldridge) were dissolved in 5 ml of iron oxide suspension in $CHCl_3$ as described above. The organic solution was added to 15 ml of distilled water pre-cooled to 0° C, and the mixture was emulsified by sonication on the ice bath. $CHCl_3$ was removed by rotavaporation at 32° C. The particles were filtered through 6 $\mu$m Whatman paper filter. The size of the nanoparticles was found to be 302 nm and remained stable for at least one week. The iron content in the nanoparticle suspension was found to be 74% with only 2.4% localized outside the nanoparticles indicating a high yield of the applied magnetite entrapment procedure. The total amount of PEI initially taken was determined in the formulation with only 13.5% localized outside the nanoparticles; the escape of PEI from the nanoparticles was slow resulting in about 30% localized outside the nanoparticles after 7 days as opposed to similarly prepared non-magnetic nanoparticles which did not include oleic acid and iron oxide in their composition.

EXAMPLE 2

PREPARATION OF NANOPARTICLES COMPRISING BIOMATERIAL

Nanoparticles were prepared as described in Example 1. DNA encoding for green fluorescent protein was used as a biomaterial. DNA in 5% glucose aqueous solution was added to nanoparticles suspended in 5% aqueous glucose solution, incubated at RT for 30 min; and gently mixed by up and down pipetting.

The nanoparticles were shown to completely bind DNA at theoretical nitrogen:phosphorus ratios above 10 and 5 for glucose 5% solution and MES 0.1 M (pH=6.5) buffer, respectively, used as complexation media.

Next particles were studied for transfection of cells in culture with and without use of external magnetic field.

Three kinds of particles were prepared at three different PEI:DNA theoretic ratios (5:1; 10:1; and 15:1).

One kind of particles denoted in Figs 5A and 5B as "magnetic NP" included magnetic nanoparticles comprising PLA, PEI, oleic acid, iron oxide coated with oleic acid and DNA (shown in Fig. 1 as magnetic NP). Serving as controls, "PEI" included particles comprising PEI and DNA and "PEI+blank NP" were particles consisting of free PEI mixed with DNA in the presence of blank PLA particles.

Next, particles were added to BAEC cells and transfection was measured in the absence and presence of external magnetic field as shown in Figs 5A and 5B respectively.

EXAMPLE 3

TRANSFECTION EFFICACY AND TOXICITY

Bovine aortic endothelial cells (BAEC) were seeded on day -1 ($2 \times 10^4$/well, on four 24-well plate). The cells were washed 2 times (2 x 1 hr) with the unsupplemented medium (DMEM) on day 0 prior to transfection. DNA stock solution (0.55 ml) was slowly added to 0.55 ml of complexants diluted to provide 0.25 g DNA per well complexed at predetermined theoretical charge ratios and left for 30 min. 0.125 ml of the unsupplemented medium was added to each well followed by 0.125 ml preparation; the cells were incubated at 37 °C, while one plate was placed at a time on the magnet (15 min) (the other kept at a distance from it). The medium was then replaced with fresh pre-warmed medium supplemented with 10% FCS. The cells were observed for transfection after 24 hr.

The nanoparticles and PEI showed comparable transfection of BAEC cells in culture with DNA encoding for green fluorescent protein when applied without use of external magnetic field (Fig 5 A), whereas the transfection efficacy of the magnetic nanoparticles applied in the presence of a permanent magnet (Fig. 5B) was substantially increased as compared to both control formulations and the magnetic nanoparticles applied in the absence of the magnet.

A similar effect of the magnetic field on the transfection efficacy was also observed in A10 cells in culture.

Notably, the magnetic NP were able to effectively transfect cells in presence of 10% and 80% serum apparently due to their protective effect against DNA enzymatic degradation, whereas practically no transfection was found when DNA:PEI complex was added to the cells in the presence of serum for the same time period.

Magnetic nanoparticles were found superior to PEI with regards to cell toxicity when applied to BAEC cells in equivalent amounts as shown in Fig. 6. Cell toxicity was measured by WST-1 assay following 4-hour incubation at 37°C.

EXAMPLE 4

USE OF SOLVENT TO CONTROL SIZE OF PARTICLE

Use of tetrahydrofuran (THF), a water-miscible solvent, in a mixture with chloform to form the organic phase allows to considerably decrease the size of the resultant nanoparticles, thus making possible to optimize the nanoparticle uptake by a given cell type without affecting the amounts of the structural components in the formulation (i.e. PLA, PEI, $Fe_3O_4$, oleic acid) and the colloidal stability of the dispersion. Therefore, one can independently control size and surface charge, which is unachievable in complexes prepared by simply combining DNA and PEI. The effect of varying the volume of THF and chloroform (the organic phase composition) on particle size and size stability (67 mg ferric chloride: 33 mg ferrous chloride, 45 mg oleic acid) was observed within 24 hours of preparation (day 0) and on the seventh day as shown is shown in Fig. 7. The total volume of the tetrahydrofuran/chloroform mixture was kept constant and equal to 6.0 ml.

The extent and stability of PEI association with magnetic NP as a function of the organic phase composition is shown in Fig. 8.

EXAMPLE 5

Preparation of water-soluble photo-activatable polymers based on poly(allylamine) Synthesis of PAA-BzPh

Synthesis of PAA-BzPh is demonstrated in Fig. 10. Poly(allylamine) (PAA) base was prepared from PAA hydrochloride (Sigma-Aldrich St. Louis, MO, MW = 70 KDa) by treatment in aqueous medium with a strong anionite Dowex G-55 followed by replacement of water by 2-propanol. A 5.1% solution of PAA base in 2-propanol (4.06 g, containing 3.65 mmol of amino groups) was diluted with $CH_2Cl_2$ (7 ml) and cooled on an ice bath. Succinoyl 4-benzoylbenzoate (Sigma-Aldrich, 236 mg, 0.73 mmol) in $CH_2Cl_2$ (12 ml) was added over a 10-min period. The mixture was stirred near 0°C for 10 min, then warmed to room temperature and acidified with concentrated HCl (0.24 ml, 2.9 mmol). The resulting suspension was dried in vacuo, resuspended in $CH_2Cl_2$, and the precipitate was filtered off. After washing with $CH_2Cl_2$ and pentane, 0.544 g of PAA-BzPh hydrochloride were obtained. A $^1H$ NMR study of this polymer (utilizing $D_2O$) indicated that 20% of polymer's amino groups were modified with 4-benzoylbenzoic residues (broad signal at 6.9-8.0 ppm). Analogously, using the calculated amount of fluorescein isothiocyanate (FITC) (Sigma-Aldrich, St. Louis, MO,) simultaneously with succinoyl 4-benzoylbenzoate in the reaction with PAA base, FITC-labeled PAA-BzPh having about 20% of 4-benzoylbenzoic residues and about 2% of the FITC label was prepared. Synthesis of PDT-BzPh

Synthesis of PDT-BzPh is shown in Fig. 10. A 5.1 % solution of PAA base in 2-propanol (2.671 g, containing 2.40 mmol of amino groups) was diluted with $CH_2Cl_2$ (5 ml) and cooled on ice. Succinoyl 4-benzoylbenzoate (145 mg, 0.45 mmol) and SPDP (Pierce Biotechnology Inc, Rockford, IL, 281 mg, 0.90 mmol) were simultaneously dissolved in $CH_2Cl_2$ (8 ml) and introduced over a 5-min period. The mixture was stirred near 0°C for 15 min, and succinic anhydride

(130 mg, 1.30 mmol) was added at once. The stirring at 0°C was continued for 0.5 h, the mixture was dried in vacuo and extracted first with ethyl acetate and then with water. The polymeric residue was dissolved in water (15 ml) with addition of $KHCO_3$ (0.3g, 3.0mmol). The solution was filtered and acidified with $H_3PO_4$ to pH of 3.5. The precipitate was filtered off, washed with water, and air-dried PDT-BzPh (488 mg) was obtained. A $^1H$ NMR study of this polymer (utilizing $D_2O$ and $K_2CO_3$ at pH 9) indicated that about 40% of 2-pyridyldithio groups and about 20% of 4-benzoylbenzoic residues were attached to the PAA backbone. The rest of amino groups was modified with 3-carboxypropionyl residues resulting from succinic anhydride.

COMPARATIVE EXAMPLE 5A

Preparation of water-soluble photo-activatable polymers based on poly(acrylic acid)

Poly(acrylic acid) can be coupled in aqueous solutions simultaneously with amino-derivatized benzophenone compound and a compound (also amino-derivatized) containing one of the reactive groups described above, particularly 2-(2-pyridyldithio)ethylamine. Water-soluble carbodiimide (EDC) can be used for such coupling (see Scheme below).

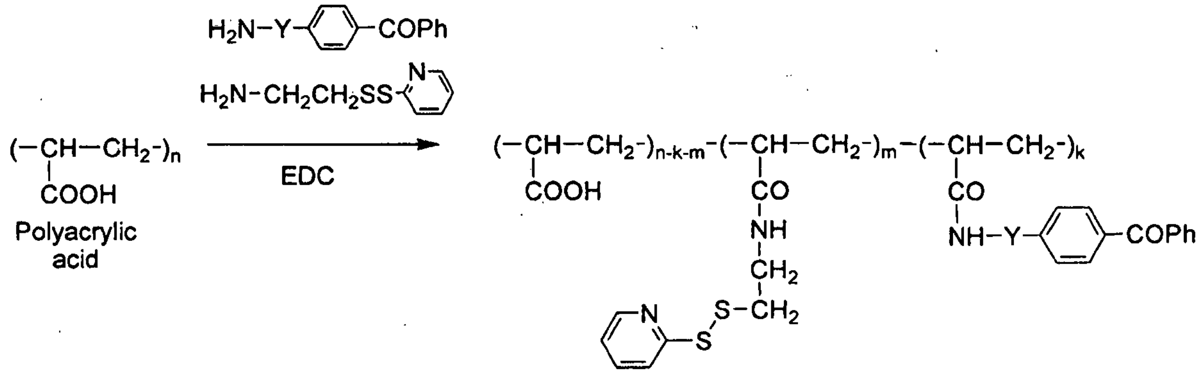


Y = any suitable spacer
EDC = 1-ethyl-3-dimethylaminopropylcarbodiimide

EXAMPLE 6

Photo-Immobilization of Polymeric Modifiers onto Matrix:

Surface amination of polymeric matrix with PAA-BzPh.

An aqueous solution (2 mg/ml) of PAA-BzPh or its FITC-labeled variant was mixed with an equal volume of a buffer containing 0.1M $NH_4OAc$ and 0.05M $NH_3$. PU Tecothane TT-1074A films or polyester (PE) fibers were immersed into the mixture for 5 - 60 min, rinsed with a 1% solution of $NH_3$ and dried on a filter paper. The polymers were irradiated under an UV-lamp (UVGL-25, long wave) for 15 - 30 min to achieve the covalent binding of modifiers to the polymer surface. Finally, the surface modified polymers were thoroughly washed with diluted (2%) HCl and water.

Modification of polymeric matrix with PDT-BzPh.

PDT-BzPh (30 mg) was dissolved in water (30 ml) by addition of $KHCO_3$ (20 mg) and acidified with a 20% solution of $KH_2PO_4$ (1 ml). PU films and PE fibers were soaked in the resulting mixture for 5 - 40 min., rinsed with 0.1 % acetic acid, dried and irradiated as above. Finally, the polymers were exhaustively washed with 0.1 M $KHCO_3$ and water.

EXAMPLE 7

Fluorescent Labeled PAA-BzPh Studies Demonstrating Attachment of Biomolecules

Fluorescence microscopy of PU films and PE fibers surface modified with FITC-labeled PAA-BzPh was conducted and confirmed the presence of the modifier bound to the polymer surfaces.

EXAMPLE 8

Cell Culture Data Demonstrating Antibody Linkage of Cy3 labeled GFP-Adenovirus PU Matrix

Surface-aminated PU films (group $NH_2$-A) were reacted with LC-sulfo-SPDP dissolved in PBS (9 mg/ml; 1 ml; 90 min). Then, the films were extensively washed in PBS and reacted in 5%BSA with anti-knob Ab (0.66 mg/ml) reduced with 1.5 mg of 2-mercaptoethylamine for 90 min at 37°C. Prior to conjugation, Ab was purified by gel filtration using a desalting column equilibrated with degassed PBS containing 10 mM EDTA. The conjugation was allowed to run for 38 hours at room temperature (RT) under mild shaking. Next, the films were washed in PBSx3 and immersed in the suspension of $10^{11}$ particles of Cy3-labeled adenovirus (Cy3-AdV-GFP) in 1.5 ml of 5% BSA/PBS. Surface aminated films that were not modified by antiknob Ab ($NH_2$-B) served as controls. Immunoconjugation was carried out for 12 hours at RT under mild shaking. Finally, the films were washed in PBS and examined under fluorescent microscope to assess tethering of Cy3-labeled adenoviruses. A uniform virus coverage of the surface was observed for the films conjugated with antiknob Ab, while the control films were virtually non-fluorescent.

PDT-BzPh-modified PU Tecothane TT-1074A films were directly modified with the reduced antiknob Ab. After washing the films (PDT-A) along with the control samples, the PDT-BzPh-modified PU samples that were not conjugated with the antiknob Ab (PDT-B), both PDT-A and PDT-B were incubated with Cy3-labeled GFP-AdV. Antibody reduction, purification and conjugation, and virus tethering were carried out according the procedures outlined above. Similar to the results obtained for the surface-aminated PU samples, a uniform fluorescent AdV layer was observed for the Ab-mediated AdV tethering, while no Cy-3-labeled AdV was bound to the surface of control films.

PE Matrix

PDT-BzPh-modified PE fibers were reacted with 2 mg of antiknob antibody reduced with 10 mg of 2-mercaptoethylamine at 37°C for 1 hour. Prior to utilization, the reduced Ab was purified using a desalting column equilibrated with degassed PBS/10 mM EDTA. The conjugation was carried out in 5% BSA/PBS for 20 hours at room temperature under mild shaking.

After PBS x 3 washing, the Ab-coupled fibers were immersed into the suspension of 5 x$10^{11}$ particles of Cy3-labeled adenovirus in 1.5 ml of 5% BSA/PBS. Immune conjugation was carried out for 14 hours at RT under mild shaking. Immobilization of Cy3-AdV on the surface of PDT-BzPh-modified PE fibers was confirmed by fluorescent microscopy.

EXAMPLE 9

Preparation of nanoparticles (NPs) Surface-Modified with PDT-BzPh

In the following example, an aqueous dispersion of NPs was prepared by emulsification-solvent evaporation and subsequently surface-modified with PDT-BzPh

D,L-polylactide (D,L-PLA, Sigma), branched poly(ethyleneimine) (PEI 25K, Aldrich) and Poloxamer 188 (Pluronic F-68, Sigma) were dissolved in 5 ml DCM HPLC grade in amounts of 300 mg, 100 mg and 50 mg, respectively.

Polylactide is a nanoparticle-forming polymer (i.e. constitutes the polymeric matrix of the nanoparticle). Poly(ethyleneimine) makes the nanoparticles cationic, capable of binding anionic substances and contributes to nanoparticles'stability during the emulsification step of their preparation; is not a requisite and can be omitted. Poloxamer 188 is a non-ionic stabilizer, which is required for nanoparticles' stability, it provides sterical stabilization.

The organic solution was emulsified in 15 ml of distilled water on ice-bath at 0°C using sonication. The solvent was removed by rotary evaporation at 35°C. The NPs were filtered through Whatman paper filter (2.5 $\mu$m cut-off). The NP size was determined by Photon Correlation Spectroscopy and was found to be 600 nm. Next, the NPs were dialyzed to remove unbound stabilizers (300 KDa cut-off MW) in distilled water at 4°C for 48 hr with several water replacements.

2 ml of preformed PLA/PEI nanoparticles were mixed with 1 ml of 0.1% aqueous solution of PDT-BzPh. 50 $\mu$l of aqueous solution of 15% $KH_2PO_4$ were added to the reaction mixture to adjust the pH to 5.5. At this pH, PDT-BzPh separates from the solution, presumably associating with the lipophilic surface of the NPs. The total amount of PDT-BzPh in the formulation (1 mg) was calculated to have a 4-fold excess over the estimated amount of the PDT-BzPh needed to establish a monolayer on the surface of PLA/PEI nanoparticles. The reaction mixture was transferred to a round-bottom flask. The flask was rotated (at 100 rpm) causing thin layer distribution of the reaction volume over the flask's walls. The hand-held UV lamp (UVGL-25, UVP) was approximated to the rotating flask, and the reaction mixture was irradiated at about 350 nm from the distance 1-2 cm for 30 min at room temperature.

According to another method of preparing particles, the BzPh-PDT polymer is included during the particle formation stage. In this variant, the BzPh-PDT acts as a particle surface stabilizer without additional colloidal stabilizers as in the previous protocol.

The organic phase obtained by dissolving 200 mg poly(D,L-lactide) (Mn = 32,000) in 5 ml chloroform, added to 15 ml of aqueous phase comprising 10 mg BzPh-PDT and 6.7 mg $KHCO_3$ and pre-cooled to 0 °C. Next, the mixture was emulsified by sonication on ice bath. Chloroform was removed by rotavaporation at 30 °C. The particles were filtered through a 1.0 μm glass fiber filter, UV-irradiated in a Petri dish for 5 min with several stirrings on a preparative UV-lamp and separated from the free BzPh-PDT by gel filtration using agarose gel with a fractionation range 25,000-2,400,000 and an exclusion limit of 4,000,000. The thiol-reactive particles thus obtained could further be used for surface modification with thiol-containing proteins, such as cysteinated D1 or reduced IgG immunoglobulins.

EXAMPLE 10

Tethering of Biomolecules to NPs Modified with DPT-BzPh PDT-BzPh -modified NPs were separated from the excess of PDT-BzPh by the dialysis against double distilled water (DDW) through the 300 kDa cut-off membrane (24 hours, 3 changes of DDW)the BzPh-PDT polymer is included during the particle formation stage, the dialysis is not necessary.A thiolated (cysteinated) form of D1 domain of the Coxsackie-Adenovirus receptor (produced by inventors according to the procedure described in Nyanguile et al, Gene Ther., 2003; 10:1362-1369) was conjugated to the thiol-reactive nanoparticles to impart adenovirus-tethering properties.

2 mg of D1 was column-desalted in 2.7 ml of degassed MES (0.01M, pH 6.5) supplemented with 10 mM EDTA and mixed with 3.3 ml of PDT-BzPh-modified nanoparticles. The coupling reaction was carried out for 14 hours at room temperature under moderate shaking. D1-derivatized nanoparticles were separated from the excess of D1 by by gel filtration using agarose gel with a fractionation range 25,000-2,400,000 and an exclusion limit of 4,000,000. Finally, 2 ml of the eluate was mixed with 125 μl of Ad-GFP (3.75 x $10^{11}$ particles). BSA was added to final 5% concentration. Immune conjugation was carried out at room temperature under mild shaking for 12 hours. 2 ml of diluted (1:3) non-modified PLA/PEI NPs were mixed with the same amount of BSA and Cy3-AdV-GFP to serve as control. Tethering of fluorescent Cy3-labeled adenovirus (AdV) to NPs was documented by fluorescent microscopy.

In another variant of this protocol, 500 μl of D1 solution (ca 4 mg/ml) were gel filtered through a desalting column (PIERCE 43243) equilibrated with degassed 0.01 M MES/10 mM EDTA (pH=6.5). The fraction between 2.5 and 4.5 ml of the eluent was collected. One ml aliquots of the desalted thiolated D1 were mixed with 1 ml of the nanoparticles. The conjugation was allowed to run for 20 hours at room temperature under argon with shaking. D1-derivatized nanoparticles were separated from the excess of D1 by gel filtration using agarose gel with a fractionation range 25,000-2,400,000 and an exclusion limit of 4,000,000. One ml of the nanoparticles was mixed with 1 ml of adenovirus suspension containing 1.48 x$10^{11}$ virus particles. The nanoparticle-virus conjugation was carried out for 2.5 hours at room temperature in presence of 5% bovine serum albumin under mild shaking.

EXAMPLE 11

Preparation of Magnetic Particles

Magnetically-responsive particles can be prepared similarly to the procedure described in Example 5A using ultrasmall magnetite/maghemite nanocrystals dispersion in chloroform instead of pure chloroform as stated in the procedure above. Such dispersion can be obtained by precipitation of aqueous ferrous chloride or its co-precipitation with ferric chloride in an alkaline aqueous solution and further stabilized with a fatty acid (See De Cuyper M, Joniau M. Magnetoliposomes. Formation and structural characterization. Eur Biophys J1988; 15:311-9; Khalafalla SE. Magnetic fluids, Chemtech 1975, Sept.: 540-7) that also imparts a degree of lipophilicity to the nanocrystal surface depending on the hydrocarbon chain length and the coating density. Such coated nanocrystals can further be extracted into or re-suspended in chloroform as well as other organic solvents, such as dichloromethane, tetrahydrofuran, etc., which can be used for polymer-based particle formulation by the methods mentioned above as in the following example:

One ml of an aqueous solution containing 65 mg $FeCl_3$ hexahydrate, 32 mg $FeCl_2$ tetrahydrate and 10 mg PLURONIC F-68 was rapidly mixed with 10 ml aqueous solution of NaOH (0.1 M). Oleic acid (100 mg) was added dropwise, and the mixture was degassed in argon. The contents were mixed and heated in a water bath for 5 min at 90 °C and cooled to room temperature. The suspension was sedimented on a Ne-B-Fe magnet for 5 min, the liquid above the precipitate was carefully aspirated, and the precipitate was mixed with 5 ml $CHCl_3$. The obtained organic dispersion of oleic acid surface-modified magnetite was added to 200 mg poly(D,L-lactide) (Mn = 32,000) and vortexed to completely dissolve the polymer, and further used as an organic phase as described in Examples 9 and 10.

While the invention has been described in detail and with reference to specific examples thereof, it will be apparent to one skilled in the art that various changes and modifications can be made therein without departing from the spirit and scope thereof.

## Claims

1. A water-soluble photo-activatable polymer comprising a polymer precursor which is poly(allylamine) and the following groups covalently attached to the poly(allylamine):

   (a) a photo-activatable group that upon irradiation is capable of forming a covalent bond between the water-soluble photo-activatable polymer and a matrix having at least one carbon;
   (b) a thiol-reactive group; and
   (c) a hydrophilic group, wherein the hydrophilic group is present in an amount sufficient to make the water-soluble photo-activatable polymer soluble in water;

   wherein the thiol-reactive group is a 2-pyridyldithio group, and the hydrophilic group is a carboxy group.

2. The water-soluble photo-activatable polymer of claim 1, wherein the polyallylamine has a molecular weight of 200 KDa to 5 KDa, in particular from 70 to 15 KDa.

3. The water-soluble photo-activatable polymer of claim 1, wherein the photo-activatable group is a member selected from the group consisting of an aryl ketone and an aryl azide.

4. The water-soluble photo-activatable polymer of claim 3, wherein the aryl ketone is a member selected from the group consisting of benzophenone and acetophenone.

5. The water-soluble photo-activatable polymer of claim 1, wherein the water-soluble polymer is represented by a formula:

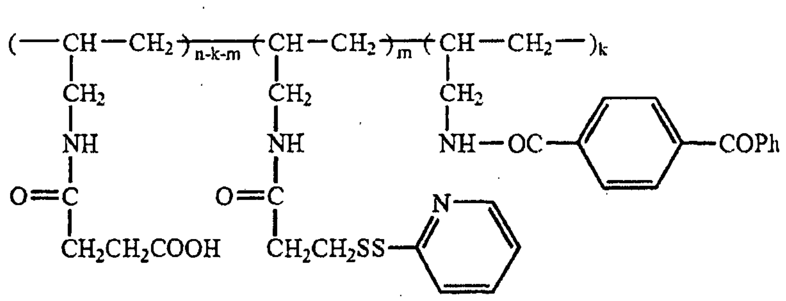

   wherein n is 50 to 2000, k is 10 to 1000, and m is 10 to 1000.

6. A process for producing the water-soluble photo-activatable polymer of claim 1, the process comprising:

   providing a polymer precursor comprising a plurality of reactive groups and a plurality of hydrophilic groups;
   providing a photo-activatable reagent; and
   reacting a first portion of the reactive groups and/or hydrophilic groups with the photo-activatable reagent.

7. The process of claim 6, wherein the first portion is from 1 % to 50% of the reactive groups and/or hydrophilic groups, in particular about 20% of the reactive groups and/or hydrophilic groups.

8. The process of claim 7, further comprising:

   providing a reagent comprising a 2-pyridyldithio group; and
   reacting a second portion of the reactive groups and/or hydrophilic groups with the reagent to obtain the water-soluble polymer.

9. The process of claim 10, wherein a sum of the first portion and the second portion is from 60% to 80%.

10. A composition comprising a monomolecular layer of the water-soluble photo-activatable polymer of claim 1 and a matrix having at least one carbon, wherein the monomolecular layer is covalently attached to the matrix by a covalent bond between the photo-activatable group and the at least one carbon.

**11.** The composition of claim 10, wherein the water-soluble photo-activatable polymer is polyallylamine having benzophenone or polyallylamine having benzophenone, a 2-pyridyldithio group, and a carboxy group.

**12.** The composition of claim 10, further comprising a biomaterial having a plurality of active groups, wherein the biomaterial is covalently attached to the monomolecular layer by covalent bonding between the active groups and thiol-reactive groups.

**13.** The composition of claim 12, wherein at least one of the active groups is a member selected from the group consisting of amine, carboxyl, hydroxyl, thiol, phenol, imidazole, and indole.

**14.** The composition of claim 12, wherein at least one of the active groups comprises thiol.

**15.** The composition of claim 12, wherein the biomaterial is a member selected from the group consisting of an antibody, a viral vector, a growth factor, a bioactive polypeptide, a polynucleotide coding for the bioactive polypeptide, a cell regulatory small molecule, a peptide, a protein, an oligonucleotide, a gene therapy agent, a gene transfection vector, a receptor, a cell, a drug, a drug delivering agent, nitric oxide, an antimicrobial agent, an antibiotic, an antimitotic, dimethyl sulfoxide, an antisecretory agent, an anti-cancer chemotherapeutic agent, steroidal and non-steroidal antiinflammatories, hormones, an extracellular matrix, a free radical scavenger, an iron chelator, an antioxidant, an imaging agent, and a radiotherapeutic agent.

**16.** The composition of claim 15, wherein the biomaterial is at least one of an anti-knob antibody, an adenovirus, a D1 domain of the Coxsackie-adenovirus receptor, insulin, an angiogenic peptide, an antiangiogenic peptide, avidin, biotin, IgG, protein A, transferrin, and a receptor for transferrin.

**17.** The composition of claim 10, wherein the matrix is a member selected from a group consisting of polyurethane, polyester, polylactic acid, polyglycolic acid, poly(lactide-co-glycolide), poly(ε-caprolactone), polyethyleneimine, polystyrene, polyamide, rubber, silicone rubber, polyacrylonitrile, polyacrylate, and polymethacrylate, poly(alpha-hydroxy acid), poly(dioxanone), poly(orthoester), poly(ether-ester), poly(lactone), polytetrafluoroethylene, organosilane, mixtures thereof and copolymers thereof.

**18.** The composition of claim 17, wherein the matrix further comprises a superparamagnetic agent.

**19.** The composition of claim 18, wherein the superparamagnetic agent is a member selected from the group consisting of magnetite and maghemite nanocrystals.

**20.** The composition of claim 10, wherein the matrix is an implantable device.

**21.** The composition of claim 20, wherein the implantable device comprises at least one member selected from the group consisting of polyurethane, polyester, polylactic acid, poly(lactide-co-glycolide), poly(ε-caprolactone), polyethyleneimine, polystyrene, polyamide, rubber, silicone rubber, polyacrylonitrile, polyacrylate, polymethacrylate, polytetrafluoroethylene, organosilane, mixtures thereof and copolymers thereof.

**22.** The composition of claim 10, wherein the matrix is a particle having a diameter of 5 nm to 10 microns.

**23.** The composition of claim 22, wherein the particle comprises at least one member selected from the group consisting of polylactic acid, polyglycolic acid, poly(lactide-co-glycolide), poly(ε-caprolactone), polyethyleneimine, poly(lactone), mixtures thereof and copolymers thereof.

**24.** A method of making the composition of claim 10, the method comprising;
providing the matrix having at least one carbon;
providing an aqueous solution of the water-soluble photo-activatable polymer having the photo-activatable group and the 2-.pyridyldithio group;
contacting the matrix with the aqueous solution; and
photo-activating the photo-activatable group by irradiation to covalently attach the water-soluble polymer via the photo-activatable group to the matrix and thereby forming the monomolecular layer of the composition.

**25.** The method of claim 24, wherein the irradiation is performed at a wavelength from 190 to 900 nm, in particular at a wavelength of 280 to 360 nm.

**26.** The method of claim 25, further comprising:

providing a biomaterial having a plurality of active groups; and
reacting the plurality of active groups with the water-soluble photo-activatable polymer to covalently attach the biomaterial to the matrix.

**27.** The method of claim 24, wherein the matrix is an implantable device.

**28.** An implantable device comprising:

a surface having a magnetizable compound and
a magnetic carrier comprising at least one of a superparamagnetic agent, magnetic- field responsive agent, and a coated magnetic-field responsive agent, wherein the magnetic carrier is attracted to the magnetizable compound by a magnetic force, provided that the magnetic carrier is a surface modified particle comprising the water-soluble photo-activated polymer of claim 1.

**29.** The device of claim 28, wherein the magnetizable compound comprises at least one of cobalt, iron, nickel, oxides and alloys thereof.

**30.** The device of claim 28, wherein the magnetizable compound comprises magnetic particles.

**31.** The device of claim 28, wherein the surface modified particle comprises a water-soluble photo-activated polymer and a biomolecule covalently attached to the water-soluble photo-activated polymer.

## Patentansprüche

**1.** Wasserlösliches, photo-aktivierbares Polymer, umfassend einen Polymer-Vorläufer, welcher Poly(allylamin) ist, und die folgenden Gruppen in kovalenter Verknüpfung mit dem Poly(allylamin):

(a) eine photo-aktivierbare Gruppe, welche nach Bestrahlung in der Lage ist, eine kovalente Bindung zwischen dem wasserlöslichen, photo-aktivierbaren Polymer und einer Matrix, die mindestens ein Kohlenstoffatom aufweist, zu bilden;
(b) eine thiol-reaktive Gruppe; und
(c) eine hydrophile Gruppe, wobei die hydrophile Gruppe in einer ausreichenden Menge vorliegt, um das wasserlösliche, photo-aktivierbare Polymer in Wasser löslich zu machen;

wobei die thiol-reaktive Gruppe eine 2-Pyridyldithio-Gruppe ist und die hydrophile Gruppe eine Carboxy-Gruppe ist.

**2.** Wasserlösliches, photo-aktivierbares Polymer nach Anspruch 1, wobei das Poly(allylamin) ein Molekulargewicht von 200 kD bis 5 kD, insbesondere von 70 bis 15 kD, aufweist.

**3.** Wasserlösliches, photo-aktivierbares Polymer nach Anspruch 1, wobei die photo-aktivierbare Gruppe ein aus der Gruppe, die aus einem Arylketon und einem Arylazid besteht, ausgewähltes Element ist.

**4.** Wasserlösliches, photo-aktivierbares Polymer nach Anspruch 3, wobei das Arylketon ein aus der Gruppe, die aus Benzophenon und Acetophenon besteht, ausgewähltes Element ist.

**5.** Wasserlösliches, photo-aktivierbares Polymer nach Anspruch 1, wobei das wasserlösliche Polymer durch eine Formel:

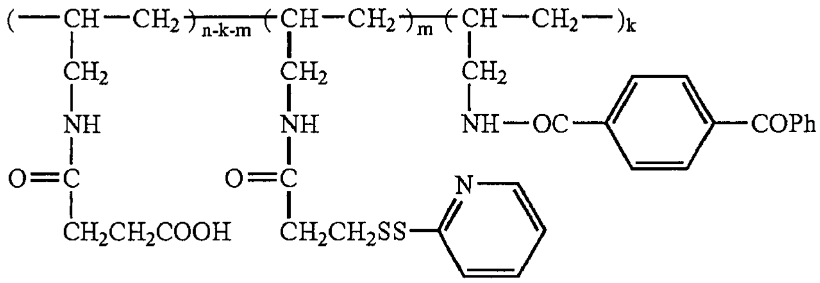

wobei n 50 bis 2000 ist, k 10 bis 1000 ist und m 10 bis 1000 ist, repräsentiert wird.

**6.** Verfahren zur Herstellung des wasserlöslichen, photo-aktivierbaren Polymers nach Anspruch 1, wobei das Verfahren umfasst:

Bereitstellen eines Polymer-Vorläufers, der eine Mehrzahl reaktiver Gruppen und eine Mehrzahl hydrophiler Gruppen umfasst;
Bereitstellen eines photo-aktivierbaren Reagenzes; und
Umsetzen eines ersten Teils der reaktiven Gruppen und/oder hydrophilen Gruppen mit dem photo-aktivierbaren Reagenz.

**7.** Verfahren nach Anspruch 6, wobei der erste Teil 1 % bis 50 % der reaktiven Gruppen und/oder hydrophilen Gruppen, insbesondere etwa 20 % der reaktiven Gruppen und/oder hydrophilen Gruppen, ausmacht.

**8.** Verfahren nach Anspruch 7, ferner umfassend:

Bereitstellen eines Reagenzes, das eine 2-Pyridyldithio-Gruppe umfasst; und
Umsetzen eines zweiten Teils der reaktiven Gruppen und/oder hydrophilen Gruppen mit dem Reagenz, um das wasserlösliche Polymer zu erhalten.

**9.** Verfahren nach Anspruch 10, wobei eine Summe des ersten Teils und des zweiten Teils 60 % bis 80 % beträgt.

**10.** Zusammensetzung, umfassend eine monomolekulare Schicht des wasserlöslichen, photo-aktivierbaren Polymers nach Anspruch 1 und eine Matrix mit mindestens einem Kohlenstoffatom, wobei die monomolekulare Schicht durch eine kovalente Bindung zwischen der photo-aktivierbaren Gruppe und dem mindestens einen Kohlenstoffatom kovalent mit der Matrix verknüpft ist.

**11.** Zusammensetzung nach Anspruch 10, wobei das wasserlösliche, photo-aktivierbare Polymer ein Polyallylamin mit einer Benzophenon-Gruppe ist oder ein Polyallylamin mit einer Benzophenon-Gruppe, einer 2-Pyridyldithio-Gruppe und einer Carboxy-Gruppe ist.

**12.** Zusammensetzung nach Anspruch 10, welche ferner ein Biomaterial mit einer Mehrzahl von aktiven Gruppen umfasst, wobei das Biomaterial durch eine kovalente Bindung zwischen den aktiven Gruppen und den thiol-reaktiven Gruppen kovalent mit der monomolekularen Schicht verknüpft ist.

**13.** Zusammensetzung nach Anspruch 12, wobei mindestens eine der aktiven Gruppen ein aus der Gruppe, die aus Amin, Carboxyl, Hydroxyl, Thiol, Phenol, Imidazol und Indol besteht, ausgewähltes Element ist.

**14.** Zusammensetzung nach Anspruch 12, wobei mindestens eine der aktiven Gruppen ein Thiol umfasst.

**15.** Zusammensetzung nach Anspruch 12, wobei das Biomaterial ein Element ist, das aus der Gruppe ausgewählt ist, welche aus einem Antikörper, einem viralen Vektor, einem Wachstumsfaktor, einem bioaktiven Polypeptid, einem Polynucleotid, kodierend für das bioaktive Polypeptid, einem kleinen zellregulatorischen Molekül, einem Peptid, einem Protein, einem Oligonucleotid, einem Gentherapeutikum, einem Gentransfektionsvektor, einem Rezeptor, einer Zelle, einem Arzneiwirkstoff, einem Arzneiwirkstoffabgabemittel, Stickstoffoxid, einem antimikrobiellen Mittel, einem Antibiotikum, einem Antimitotikum, Dimethylsulfoxid, einem antisekretorischen Mittel, einem Chemotherapeutikum gegen Krebs, steroidalen und nicht-steroidalen antiinflammatorischen Mitteln, Hormonen, einer extrazellularen Matrix, einem Radikalfänger, einem Eisenchelator, einem Antioxidationsmittel, einem Bildgebungsmittel und einem Radiotherapeutikum besteht.

**16.** Zusammensetzung nach Anspruch 15, wobei das Biomaterial mindestens eines von einem Anti-knob-Antikörper, einem Adenovirus, einer D1-Domäne des Coxsackie-Adenovirus-Rezeptors, Insulin, einem angiogenen Peptid, einem antiangiogenen Peptid, Avidin, Biotin, IgG, Protein A, Transferrin und einem Rezeptor für Transferrin ist.

**17.** Zusammensetzung nach Anspruch 10, wobei die Matrix ein aus einer Gruppe, die aus Polyurethan, Polyester, Polymilchsäure, Polyglycolsäure, Poly(lactid-co-glycolid), Poly-(ε-caprolacton), Polyethylenimin, Polystyrol, Polyamid, Kautschuk, Silikonkautschuk, Polyacrylnitril, Polyacrylat und Polymethacrylat, Poly(alpha-hydroxysäure), Poly(dioxanon), Poly(orthoester), Poly(ether-ester), Poly(lacton), Polytetrafluoroethylen, Organosilan, Mischungen davon und Copolymeren davon besteht, ausgewähltes Element ist.

**18.** Zusammensetzung nach Anspruch 17, wobei die Matrix ferner ein superparamagnetisches Agens umfasst.

**19.** Zusammensetzung nach Anspruch 18, wobei das superparamagnetische Agens ein aus der Gruppe, die aus Magnetit- und Maghemit-Nanokristallen besteht, ausgewähltes Element ist.

**20.** Zusammensetzung nach Anspruch 10, wobei die Matrix eine implantierbare Vorrichtung ist.

**21.** Zusammensetzung nach Anspruch 20, wobei die implantierbare Vorrichtung mindestens ein Element umfasst, welches aus der Gruppe, die aus Polyurethan, Polyester, Polymilchsäure, Poly(lactid-co-glycolid), Poly(ε-caprolacton), Polyethylenimin, Polystyrol, Polyamid, Kautschuk, Silikonkautschuk, Polyacrylnitril, Polyacrylat, Polymethacrylat, Polytetrafluoroethylen, Organosilan, Mischungen davon und Copolymeren davon besteht, ausgewählt ist.

**22.** Zusammensetzung nach Anspruch 10, wobei die Matrix ein Partikel mit einem Durchmesser von 5 nm bis 10 μm ist.

**23.** Zusammensetzung nach Anspruch 22, wobei das Partikel mindestens ein aus der Gruppe, die aus Polymilchsäure, Polyglycolsäure, Poly(lactid-co-glycolid), Poly(ε-capro-lacton), Polyethylenimin, Poly(lacton), Mischungen davon und Copolymeren davon besteht, ausgewähltes Element umfasst.

**24.** Verfahren zur Herstellung der Zusammensetzung nach Anspruch 10, wobei das Verfahren umfasst:

Bereitstellen der Matrix mit mindestens einem Kohlenstoffatom;
Bereitstellen einer wässrigen Lösung des wasserlöslichen, photo-aktivierbaren Polymers mit der photo-aktivierbaren Gruppe und der 2-Pyridyldithio-Gruppe;
Kontaktieren der Matrix mit der wässrigen Lösung; und
Photo-Aktivieren der photo-aktivierbaren Gruppe durch Bestrahlung, um das wasserlösliche Polymer über die photo-aktivierbare Gruppe mit der Matrix kovalent zu verknüpfen und dadurch die monomolekulare Schicht der Zusammensetzung zu bilden.

**25.** Verfahren nach Anspruch 24, wobei die Bestrahlung bei einer Wellenlänge von 190 bis 900 nm, insbesondere bei einer Wellenlänge von 280 bis 360 nm, durchgeführt wird.

**26.** Verfahren nach Anspruch 25, ferner umfassend:

Bereitstellen eines Biomaterials mit einer Mehrzahl von aktiven Gruppen; und
Umsetzen der Mehrzahl von aktiven Gruppen mit dem wasserlöslichen, photo-aktivierbaren Polymer, um das Biomaterial kovalent mit der Matrix zu verknüpfen.

**27.** Verfahren nach Anspruch 24, wobei die Matrix eine implantierbare Vorrichtung ist.

**28.** Eine implantierbare Vorrichtung, umfassend:

eine Oberfläche mit einer magnetisierbaren Verbindung und
einen magnetischen Träger, der mindestens eines von einem superparamagnetischen Agens, einem auf ein Magnetfeld ansprechenden Agens und einem auf ein Magnetfeld ansprechenden beschichteten Agens umfasst,

wobei der magnetische Träger von der magnetisierbaren Verbindung durch eine magnetische Kraft angezogen wird, mit der Maßgabe, dass der magnetische Träger ein oberflächenmodifiziertes Partikel ist, welches das wasserlösliche, photo-aktivierte Polymer von Anspruch 1 umfasst.

**29.** Vorrichtung nach Anspruch 28, wobei die magnetisierbare Verbindung mindestens eines von Kobalt, Eisen, Nickel, Oxiden und Legierungen davon umfasst.

**30.** Vorrichtung nach Anspruch 28, wobei die magnetisierbare Verbindung magnetische Partikel umfasst.

**31.** Verfahren nach Anspruch 28, wobei das oberflächenmodifizierte Partikel ein wasserlösliches, photo-aktivierbares Polymer und ein Biomolekül in kovalenter Verknüpfung mit dem wasserlöslichen, photo-aktivierbaren Polymer umfasst.

**Revendications**

**1.** Polymère photoactivable hydrosoluble comprenant un précurseur de polymère qui est une polyallylamine et les groupes suivants attachés de manière covalente à la polyallylamine :

(a) un groupe photoactivable qui lors du rayonnement est capable de former une liaison covalente entre le polymère photoactivable hydrosoluble et une matrice ayant au moins un carbone ;
(b) un groupe thiol réactif ; et
(c) un groupe hydrophile, le groupe hydrophile étant présent en une quantité suffisante pour rendre soluble dans l'eau le polymère photoactivable hydrosoluble ;

le groupe thiol réactif étant un groupe 2-pyridyldithio et le groupe hydrophile étant un groupe carboxy.

**2.** Polymère photoactivable hydrosoluble selon la revendication 1, dans lequel la polyallylamine a un poids moléculaire de 200 KDa à 5 KDa, en particulier de 70 à 15 KDa.

**3.** Polymère photoactivable hydrosoluble selon la revendication 1, dans lequel le groupe photoactivable est un élément choisi dans le groupe constitué par une arylcétone et un arylazide.

**4.** Polymère photoactivable hydrosoluble selon la revendication 3, dans lequel l'arylcétone est un élément choisi dans le groupe constitué par la benzophénone et l'acétophénone.

**5.** Polymère photoactivable hydrosoluble selon la revendication 1, dans lequel le polymère hydrosoluble est représenté par une formule :

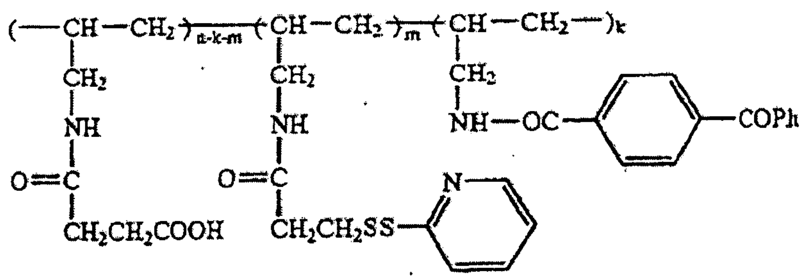

dans laquelle n vaut de 50 à 2000, k vaut de 10 à 1000 et m vaut de 10 à 1000.

**6.** Procédé de production du polymère photoactivable hydrosoluble selon la revendication 1, le procédé consistant à :

utiliser un précurseur de polymère comprenant une pluralité de groupes réactifs et une pluralité de groupes hydrophiles ;
utiliser un réactif photoactivable ; et

faire réagir une première partie des groupes réactifs et/ou des groupes hydrophiles avec le réactif photoactivable.

7. Procédé selon la revendication 6, dans lequel la première partie est de 1 % à 50 % des groupes réactifs et/ou des groupes hydrophiles, en particulier environ 20 % des groupes réactifs et/ou des groupes hydrophiles.

8. Procédé selon la revendication 7, consistant en outre à :

   utiliser un réactif comprenant un groupe 2-pyridyldithio ; et
   faire réagir une seconde partie des groupes réactifs et/ou des groupes hydrophiles avec le réactif pour obtenir le polymère hydrosoluble.

9. Procédé selon la revendication 10, dans lequel une somme de la première partie et de la seconde partie est de 60 % à 80 %.

10. Composition comprenant une couche monomoléculaire du polymère photoactivable hydrosoluble selon la revendication 1 et une matrice ayant au moins un carbone, la couche monomoléculaire étant attachée de manière covalente à la matrice par une liaison covalente entre le groupe photoactivable et l'au moins un carbone.

11. Composition selon la revendication 10, dans laquelle le polymère photoactivable hydrosoluble est une polyallylamine ayant une benzophénone ou une polyallylamine ayant une benzophénone, un groupe 2-pyridyldithio et un groupe carboxy.

12. Composition selon la revendication 10, comprenant en outre un biomatériau ayant une pluralité de groupes actifs, le biomatériau étant attaché de manière covalente à la couche monomoléculaire par une liaison covalente entre les groupes actifs et les groupes thio réactifs.

13. Composition selon la revendication 12, dans laquelle au moins un des groupes actifs est un élément choisi dans le groupe constitué par une amine, un carboxyle, un hydroxyle, un thiol, un phénol, un imidazole et un indole.

14. Composition selon la revendication 12, dans laquelle au moins un des groupes actifs comprend un thiol.

15. Composition selon la revendication 12, dans laquelle le biomatériau est un élément choisi dans le groupe constitué par un anticorps, un vecteur viral, un facteur de croissance, un polypeptide bioactif, un polynucléotide codant pour le polypeptide bioactif, une petite molécule de régulation cellulaire, un peptide, une protéine, un oligonucléotide, un agent de thérapie génique, un agent de transfection génique, un récepteur, une cellule, un médicament, un agent de délivrance de médicament, de l'oxyde nitrique, un agent antimicrobien, un antibiotique, un antimitotique, le diméthylsulfoxyde, un agent antisécrétoire, un agent chimiothérapeutique anticancéreux, des anti-inflammatoires stéroïdiens et non stéroïdiens, des hormones, une matrice extracellulaire, un piégeur de radicaux libres, un chélateur du fer, un antioxydant, un agent d'imagerie et un agent de radiothérapie.

16. Composition selon la revendication 15, dans laquelle le biomatériau est au moins un anticorps anti-knob, un adénovirus, un domaine D1 du récepteur de l'adénovirus Coxsackie, l'insuline, un peptide angiogénique, un peptide antiangiogénique, l'avidine, la biotine, l'IgG, la protéine A, la transferrine et un récepteur pour la transferrine.

17. Composition selon la revendication 10, dans laquelle la matrice est un élément choisi dans un groupe constitué par un polyuréthane, un polyester, un acide polylactique, un acide polyglycolique, un poly(lactide-co-glycolide), une poly($\varepsilon$-caprolactone), une polyéthylèneimine, un polystyrène, un polyamide, un caoutchouc, un caoutchouc de silicone, un polyacrylonitrile, un polyacrylate et un polyméthacrylate, un poly(alpha-hydroxyacide), une poly(dioxanone), un poly(orthoester), un poly(éther-ester), une poly(lactone), un polytétrafluoroéthylène, un organosilane, des mélanges de ceux-ci et des copolymères de ceux-ci.

18. Composition selon la revendication 17, dans laquelle la matrice comprend en outre un agent superparamagnétique.

19. Composition selon la revendication 18, dans laquelle l'agent superparamagnétique est un élément choisi dans le groupe constitué par les nanocristaux de magnétite et de maghémite.

20. Composition selon la revendication 10, dans laquelle la matrice est un dispositif implantable.

**21.** Composition selon la revendication 20, dans laquelle le dispositif implantable comprend au moins un élément choisi parmi un polyuréthane, un polyester, un acide polylactique, un poly(lactide-co-glycolide), une poly(ε-caprolactone), une polyéthylèneimine, un polystyrène, un polyamide, un caoutchouc, un caoutchouc de silicone, un polyacrylonitrile, un polyacrylate, un polyméthacrylate, un polytétrafluoroéthylène, un organosilane, des mélanges de ceux-ci et des copolymères de ceux-ci.

**22.** Composition selon la revendication 10, dans laquelle la matrice est une particule ayant un diamètre de 5 nm à 10 μm.

**23.** Composition selon la revendication 22, dans laquelle la particule comprend au moins un élément choisi dans le groupe constitué par un acide polylactique, un acide polyglycolique, un poly(lactide-co-glycolide), une poly(ε-caprolactone), une polyéthylèneimine, une poly(lactone), des mélanges de ceux-ci et des copolymères de ceux-ci.

**24.** Procédé de préparation de la composition selon la revendication 10, le procédé consistant à :

utiliser la matrice ayant au moins un carbone ;
utiliser une solution aqueuse du polymère photoactivable hydrosoluble ayant le groupe photoactivable et le groupe 2-pyridyldithio ;
mettre en contact la matrice avec la solution aqueuse ; et
photoactiver le groupe photoactivable par rayonnement pour attacher de manière covalente le polymère hydrosoluble, par le biais du groupe photoactivable, à la matrice et former ainsi la couche monomoléculaire de la composition.

**25.** Procédé selon la revendication 24, dans lequel le rayonnement est réalisé à une longueur d'onde de 190 à 900 nm, en particulier à une longueur d'onde de 280 à 360 nm.

**26.** Procédé selon la revendication 25, consistant en outre à :

utiliser un biomatériau ayant une pluralité de groupes actifs ; et
faire réagir la pluralité de groupes actifs avec le polymère photoactivable hydrosoluble pour attacher de manière covalente le biomatériau à la matrice.

**27.** Procédé selon la revendication 24, dans lequel la matrice est un dispositif implantable.

**28.** Dispositif implantable comprenant :

une surface ayant un composé magnétisable, et
un véhicule magnétique comprenant au moins un d'un agent superparamagnétique, d'un agent sensible au champ magnétique et d'un agent sensible au champ magnétique enduit, le véhicule magnétique étant attaché au composé magnétisable par une force magnétique, à condition que le véhicule magnétique soit une particule superficiellement modifiée comprenant le polymère photoactivé hydrosoluble selon la revendication 1.

**29.** Dispositif selon la revendication 28, dans lequel le composé magnétisable comprend au moins un élément parmi le cobalt, le fer, le nickel, les oxydes et les alliages de ceux-ci.

**30.** Dispositif selon la revendication 28, dans lequel le composé magnétisable comprend des particules magnétiques.

**31.** Dispositif selon la revendication 28, dans lequel la particule superficiellement modifiée comprend un polymère photoactivé hydrosoluble et une biomolécule attachée de manière covalente au polymère photoactivé hydrosoluble.

FIG. 1A

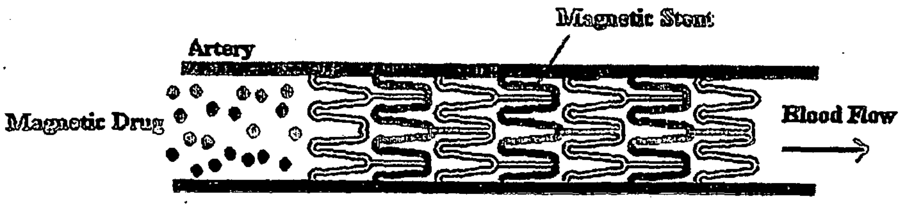


FIG. 1B

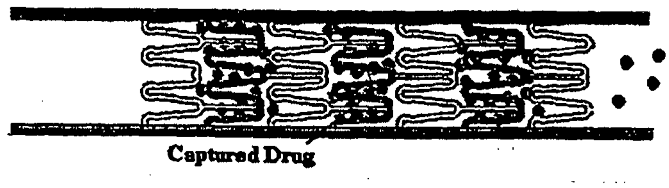


FIG. 2A

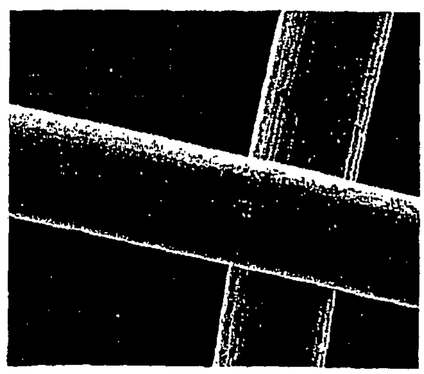

FIG. 2B

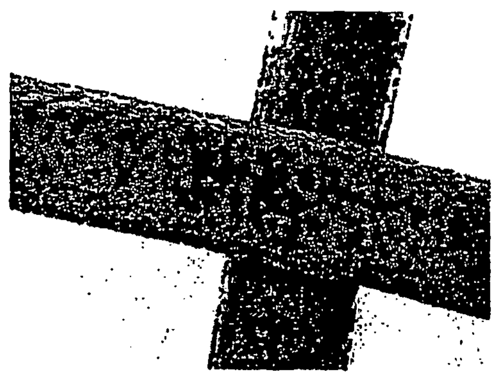

FIG. 3

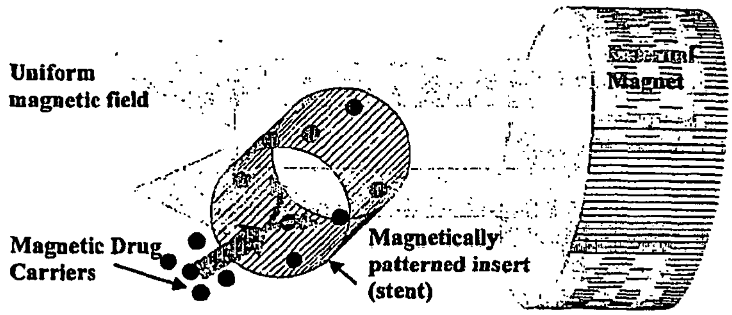


FIG. 4A

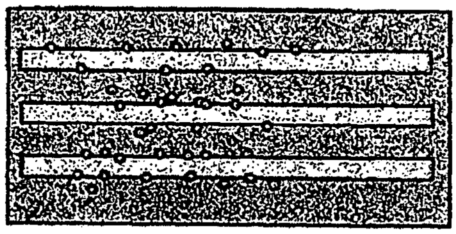

FIG. 4B

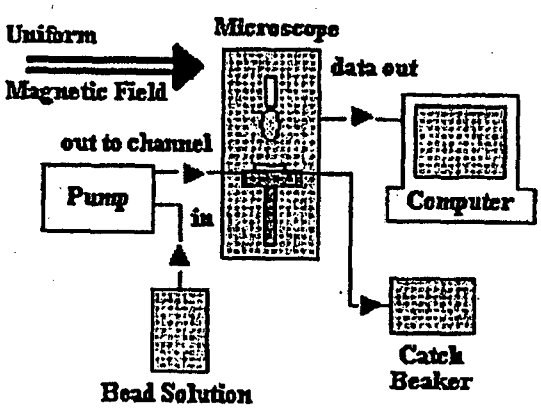

FIG. 5A

without magnetic field exposure

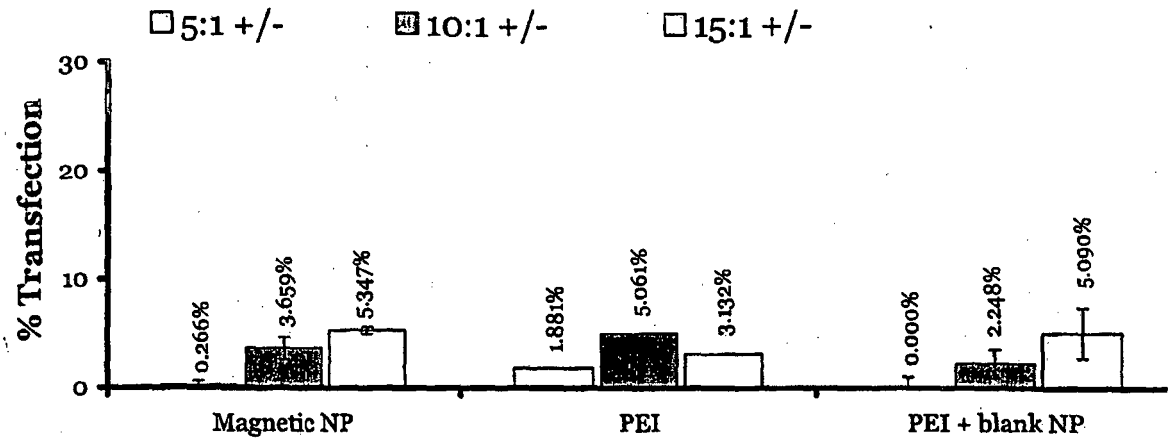


FIG. 5B

with magnetic field exposure

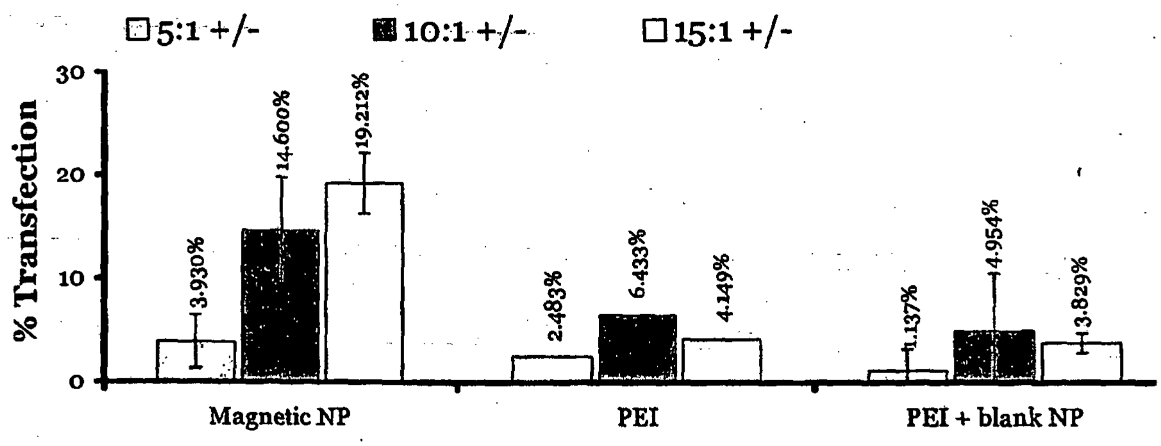

FIG. 6

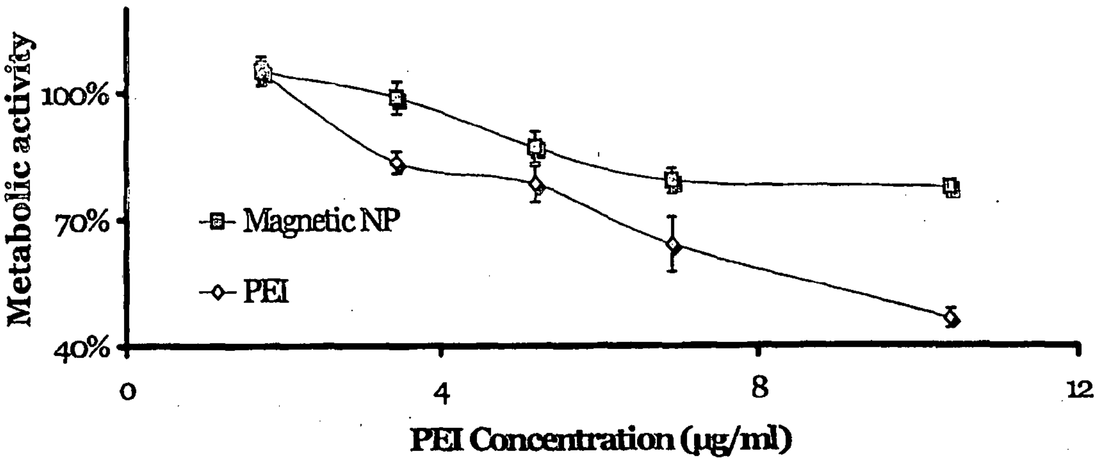
Metabolic activity
100%
70%
40%
Magnetic NP
PEI
0
4
8
12
PEI Concentration (µg/ml)

FIG. 7

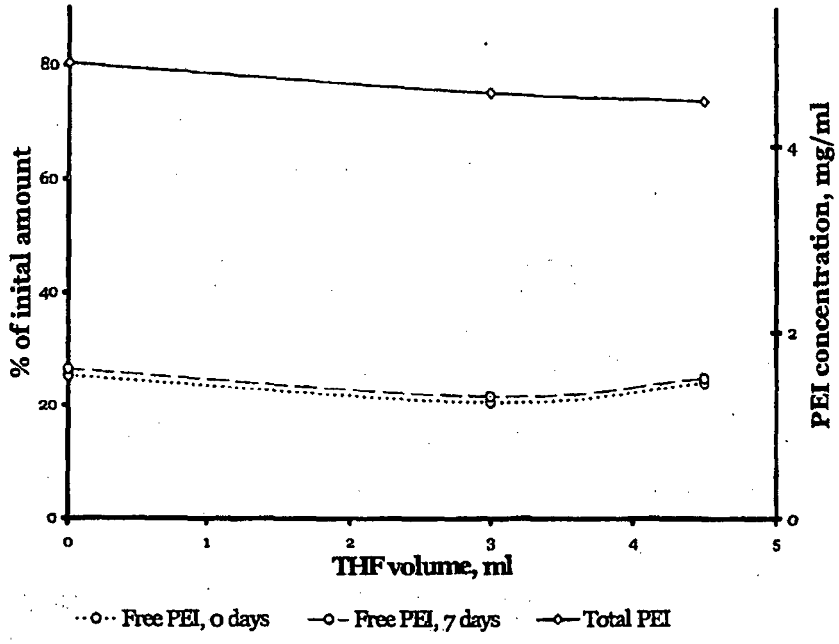
% of inital amount
PEI concentration, mg/ml
THF volume, ml
0
1
2
3
4
5
20
40
60
80
0
2
4
Free PEI, 0 days
Free PEI, 7 days
Total PEI

FIG. 8

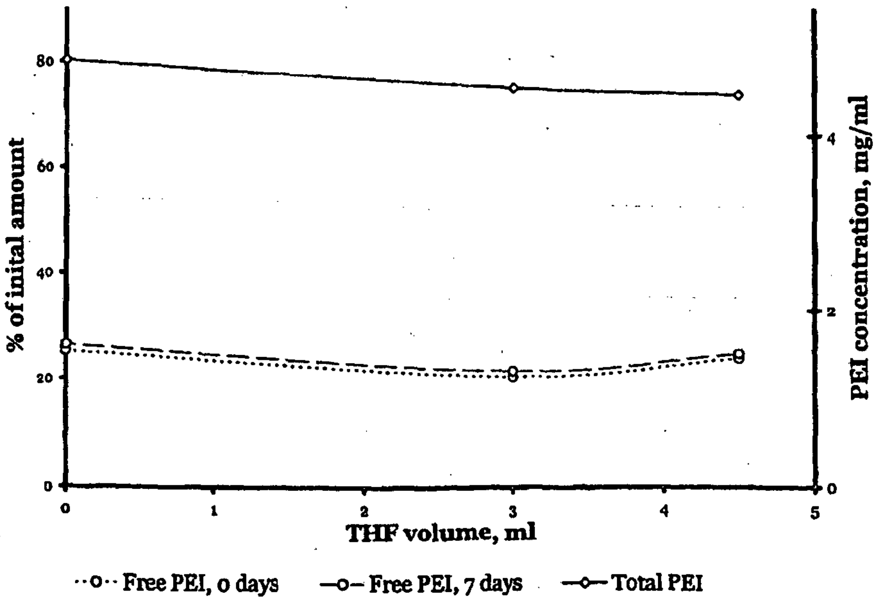
% of inital amount
PEI concentration, mg/ml
THF volume, ml
0
1
2
3
4
5
20
40
60
80
0
2
4
Free PEI, 0 days
Free PEI, 7 days
Total PEI

FIG. 9

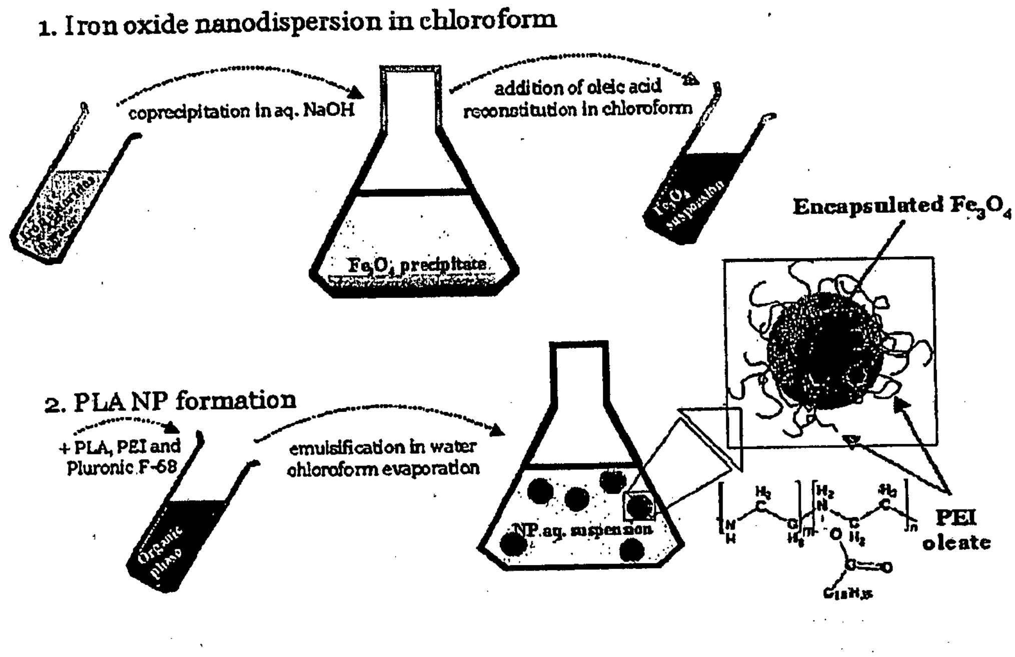
1. Iron oxide nanodispersion in chloroform
coprecipitation in aq. NaOH
addition of oleic acid
reconstitution in chloroform
Fe3O4 precipitate
Fe3O4 suspension
Encapsulated Fe3O4
2. PLA NP formation
+ PLA, PEI and
Pluronic F-68
emulsification in water
chloroform evaporation
Organic phase
NP aq. suspension
PEI
oleate

FIG. 10

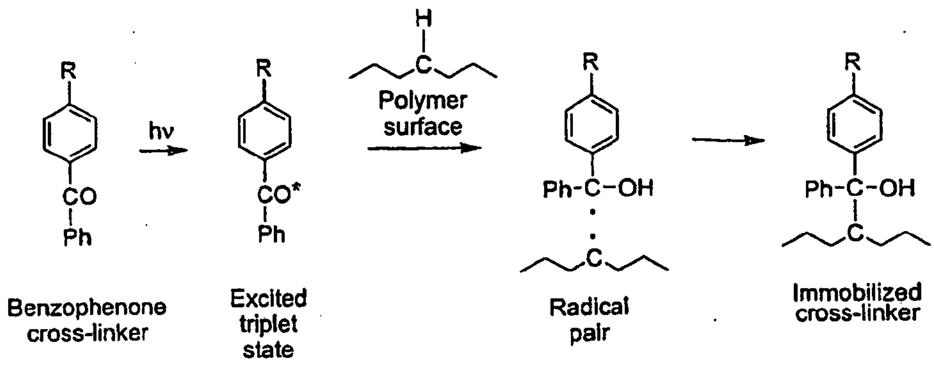
hν
Polymer surface
Benzophenone cross-linker
Excited triplet state
Radical pair
Immobilized cross-linker

FIG. 11

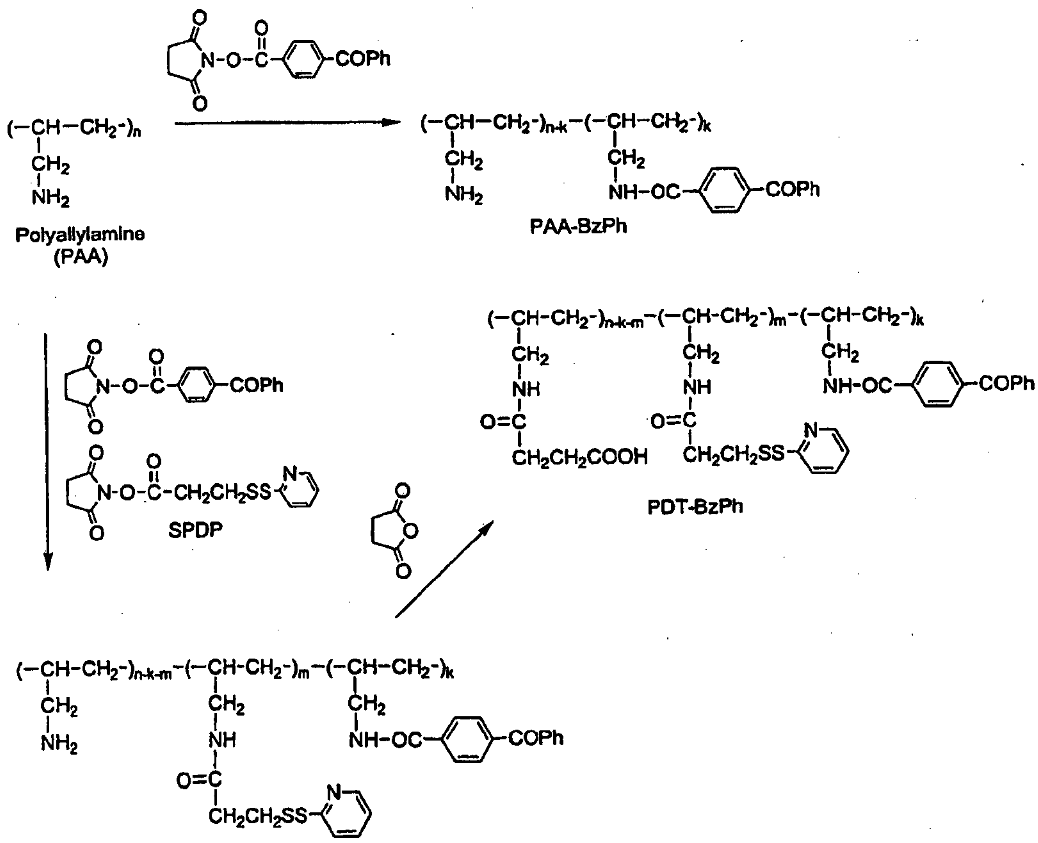
Polyallylamine (PAA)
PAA-BzPh
SPDP
PDT-BzPh

FIG. 12

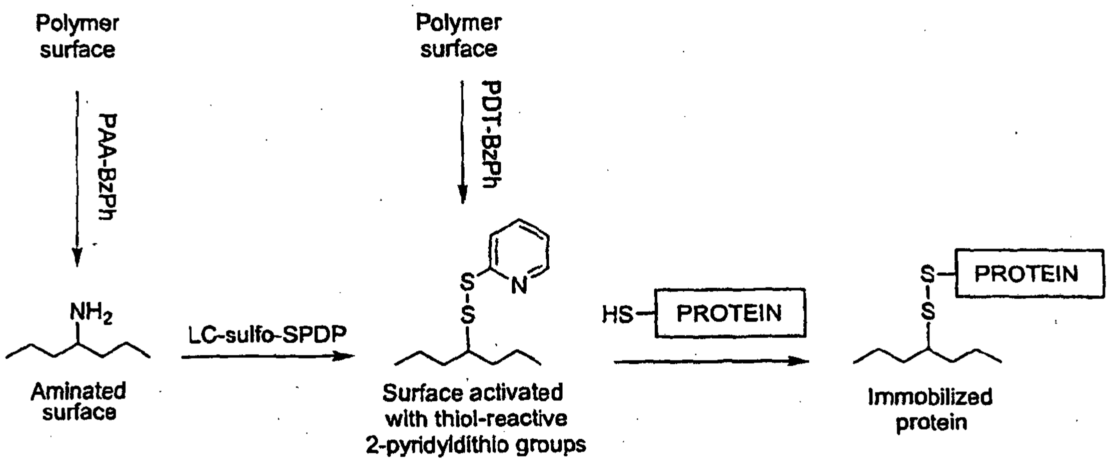


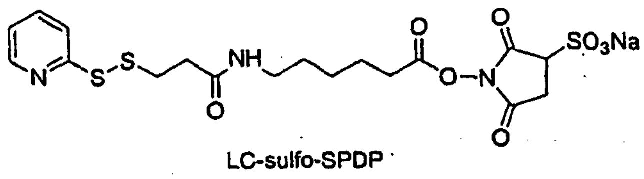

LC-sulfo-SPDP

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 4247406 A
- US 5129877 A
- US 6375606 B
- US 6315709 B
- US 6296604 B
- US 6364823 B
- US 5921244 A, Chen
- US 5916539 A, Pilgrimm
- US 5071909 A, Pappin
- US 3959078 A
- US 5512329 A
- US 5741551 A, Guire
- US 5637460 A, Swan


### Non-patent literature cited in the description


- **REGAR et al.** *Br. Med. Bull.,* 2001, vol. 59, 227-48
- **HEHRLEIN et al.** *Basic Res. Cardiol.,* 2002, vol. 97, 417-23
- **GERSHLICK et al.** *Atherosclerosis,* 2002, vol. 160, 259-71
- **GARAS et al.** *Pharmacology and Therapeutics,* 2001, vol. 92, 165-78
- **GARAS, S.M. et al.** *Pharmacology and Therapeutics,* 2001, vol. 92, 165-178
- **FRICKER.** *J. Drug Discovery Today,* 2001, vol. 6, 1135-7
- **HEHRLEIN et al.** *Basic Re. Cardiol.,* 2002, vol. 97, 417-23
- **GARAS et al.** *Pharmacology and Therapeutics,* vol. 200192, 165-78
- **SCHWARTZ et al.** *Circulation,* 2002, vol. 106, 1867-73
- **HERR, H.** *J. of Rehab. Res. and Devel.,* 2002, vol. 39 (3), 11-12
- **GOODWIN, S.** *J. of Magnetism and Magnetic Materials,* 1999, vol. 194, 209-217
- **LIU et al.** *J. of Magnetism and Magnetic Materials,* 2001, vol. 225, 209-217
- **SHENG et al.** *J. of Magnetism and Magnetic Materials,* 1999, vol. 194, 167-175
- **FORBES et al.** *Abstract and Poster Presentation at the 6th Annual New Jersey Symposium on Biomaterials,* 17 October 2002
- **Z. FORBES ; B.B. YELLEN ; G. FRIEDMAN ; K. BARBEE.** *IEEE Trans. Magn,* 2003, vol. 39 (5), 3372-3377
- **QUINTANAR-GUERRERO et al.** Preparation techniques and mechanisms of formation of biodegradable nanoparticles from preformed polymers. *Drug Dev Ind Pharm,* 1998, vol. 24, 1113-28
- **KUMAR MNR.** Nano and microparticles as controlled drug delivery devices. *J Pharm Pharmaceut Sci,* 2000, vol. 3, 234-58
- **PLANK et al.** Enhancing and targeting nucleic acid delivery by magnetic force. *Expert Opin Biol Ther,* 2003, vol. 3, 745-58
- **PLANK et al.** The magnetofection method: using magnetic force to enhance gene delivery. *Biol Chem.,* 2003, vol. 384, 737-47
- **SCHERER et al.** Magnetofection: enhancing and targeting gene delivery by magnetic force in vitro and in vivo. *Gene Ther.,* 2002, vol. 9, 102-9
- Magnetic Granules: A Novel System for Specific Drug Delivery to Esophageal Mucosa in Oral Administration. *Int'l. J. of Pharmaceutics,* 1990, vol. 61, 109-117
- Synthesis and Characterization of Poly(ethyl-2-cyanoacrylate) Nanoparticles with a Magnetic Core. *J of Controlled Release,* 2001, vol. 77, 309-321
- Synthesis and Characterization of Spherical Magnetite/Biodegradable Polymer Composite Particles. *J. of Colloid and Interface Science,* 2001, vol. 240, 40-47
- Magnetofection: enhancing and targeting gene delivery with superparamagnetic nanoparticles and magnetic fields. *J Liposome Res,* 2003, vol. 13, 29-32
- **SCHERER E.** Magnetofection: enhancing and targeting gene delivery by magnetic force in vitro and in vivo. *Gene Ther.,* 2002, vol. 9, 102-9
- **KROTZ et al.** Magnetofection potentiates gene delivery to cultured endothelial cells. *J Vasc Res.,* 2003, vol. 40 (5), 425-434
- **KROTZ et al.** Magnetofection-A highly efficient tool for antisense oligonucleotide delivery in vitro and in vivo. *Mol Ther.,* 2003, vol. 7, 700-10

- Cytotoxicity of Magnetite-Loaded Polylactide, Polylactide/Glycolide Particles and Solid Lipid Nanoparticles. *Int'l. J. of Pharmaceutics,* 1996, vol. 138, 85-94
- Development and Characterization of Solid Lipid Nanoparticles Loaded with Magnetite. *Int'l. J. of Pharmaceutics,* 2002, vol. 233, 149-157
- Magnetoliposomes. Formation and structural characterization. *Eur Biophys J,* 1988, vol. 15, 311-319
- Elaboration of Poly(ethyleneimine) Coated Poly(D, L-lactic acid) Particles. Effect of Ionic Strength on the Surface Properties and DNA Binding Capabilities. *Colloids and Surfaces B: Bioninterfaces,* 2003, vol. 32, 293-305
- Development of a Novel Gene Delivery Scaffold Utilizing Colloidal Gold-Polyethylenimine Conjugates for DNA Condensation. *Gene Therapy,* 2003, vol. 10, 1882-1890
- **WISE D L.** Encyclopedic handbook of biomaterials and bioengineering, Part A: materials. Marcel Dekker Inc, 1995, 895-926
- **WETZELS et al.** Photoimmobilization of poly(N-vinylpyrrolidone) as a means to improve haemocompatibility of polyurethane biomaterials. *Biomaterials,* 1999, vol. 20, 1879-87
- **MCCLUNG et al.** Lysine-derivatized polyurethane as a clot lysing surface: conversion of absorbed plasminogen to plasmin and clot lysis in vitro. *Biomaterials,* 2001, vol. 22, 1919-24
- **ALDENHOFF et al.** Photo-immobilization of dipyridamole (PERSANTIN®) at the surface of polyurethane biomaterials: reduction of in vitro trombogenicity. *Biomaterials,* 1997, vol. 18, 167-72
- **KUIJPENS et al.** Immobilization of theophylline on medical-grade polyurethane inhibits surface-induced activation of blood platelets. *J. Am. Chem. Soc.,* 1995, vol. 117, 8691-8697
- **CHEN et al.** Biomaterials. Elsevier Science Publishers BV, 2001, vol. 22, 2453-2457
- **GREG T. HERMANSON.** Bioconjugate Techniques. Academic Press, 1996
- Nanoparticles: preparation and characterization. **COUVREUR P et al.** Microencapsulation. Methods and industrial applications. Marcel Dekker, 1996, vol. 73, 183-211
- **KUMAR MNR.** Nano- and microparticles as controlled drug delivery devices. *J Pharm Pharmaceut Sci,* 2000, vol. 3, 234-58
- **ITO R ; MACHIDA Y ; SANNAN T ; NAGAI TU.** hwscsaBTW-B-Gddaaca. Magnetic granules: a novel system for specific drug delivery to esophageal mucosa in oral administration. *Int J Pharm,* 1990, vol. 61, 109-117
- **SEGRE, G. ; SILBERBERG, A.** *J. Fluid Mech,* 1962, vol. 14, 136
- **SEGRE, G ; SILBERBERG, A.** *J. Fluid Mech.,* 1962, vol. 14, 136
- **FORBES ZG ; YELLEN BB ; BARBEE KA ; FRIEDMAN G.** An Approach to Targeted Drug Delivery Based on Uniform Magnetic Fields. *IEEE Transactions on Magnetics,* 2003, vol. 39 (5), 3372-3377
- **GOSSELIN et al.** Efficient gene transfer using reversibly cross-linked low molecular weight polyethylenimine. *Bioconjug Chem.,* November 2001, vol. 12 (6), 989-94
- **FORREST et al.** A degradable polyethylenimine derivative with low toxicity for highly efficient gene delivery. *Bioconjug Chem.,* September 2003, vol. 14 (5), 934-40
- **HYEON T.** Chemical Synthesis of Magnetic Nanoparticles. The Royal Society of Chemistry, 2003
- *Chem. Commun.,* 2003, 927-934
- **KHALAFALLA SE.** Magnetic fluids. *Chemtech,* September 1975, 540-547
- **SAMBROOK et al.** MOLECULAR CLONING: A LABORATORY MANUAL. Cold Spring Harbor Laboratory, 1989
- **NYANGUILE et al.** *Gene Ther.,* 2003, vol. 10, 1362-1369
- **DE CUYPER M ; JONIAU M.** Magnetoliposomes. Formation and structural characterization. *Eur Biophys J,* 1988, vol. 15, 311-9
- **KHALAFALLA SE.** Magnetic fluids. *Chemtech,* September 1975, 540-7